# EUROPEAN PATENT APPLICATION

(11) **EP 1 300 403 A1**
(43) Date of publication of application: **09.04.2003**
(21) Application number: 01402551.4
(22) Date of filing: 02.10.2001
(51) Int. Cl.: C07D 311/00, A61K 31/35

(54) **Process for the manufacture of hypoxyxylerone derivatives**

(71) Applicant: Aventis Pharma S.A., 92160 Antony (FR)
(72) Inventor: Gimbert, Yves, 38470 Vinay (FR); Chevenier, Emmanuel, Saint Forgeux Lespinasse (FR); Green, Andrew E., 38410 Saint Martin D'Uriage (FR); Massadier, Christine, 42170 Saint Just, Saint Rambert (FR); Piettre, Arnaud, 02000 Laon (FR)
(74) Representative: Le Pennec, Magali

(57) **Abstract**

The present invention relates to the total synthesis of hypoxyxylerone derivatives (formula I) and their biological activities.

R1-R5 are as described in the description.

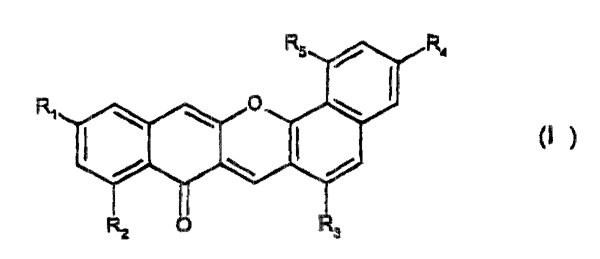

## Description

The present invention is concerned primarily with processes for the preparation of hypoxyxylerone derivatives.

Hypoxyxylerone is a green pigment extracted from the fungus hypoxylon fragiform [see "Hypoxyxylerone, a Novel Green Pigment from the Fungus Hypoxylon fragifrom", published in the Journal of the Chemical Society, Chem. Commun, (1991), 1009-10]. Hypoxyxylerone has the following formula :

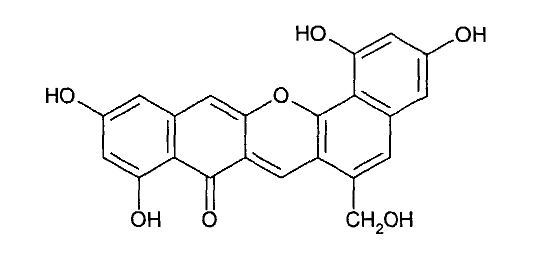

Neither its biological activity nor its synthesis nor those of derivatives have previously been described.

The present invention addresses both aspects :
- the total synthesis of hypoxyxylerone and its derivatives
- the biological activity of hypoxyxylerone and its derivatives

The chemical synthesis can be effected by two different processes :
- one by condensation of a naphthalene alcohol with a naphthalene acid, followed by closure of the fifth ring
- the other through condensation of an alkoxycarbonylmethyl benzoic acid derivative (VI) with a naphthalene-2 yl ethanone (VII). 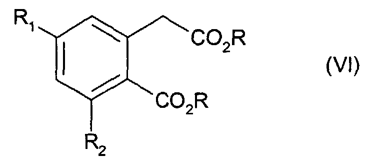 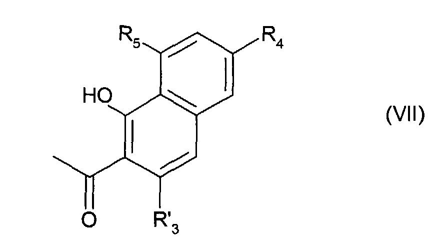
The first object of the present invention is related to a process of manufacture of hypoxyxylerone derivatives of formula (Ia), 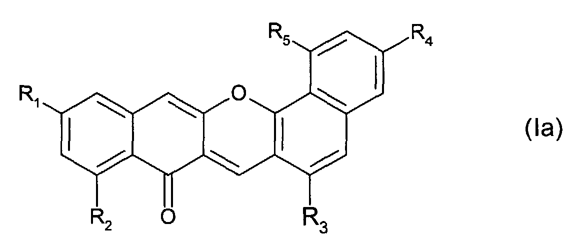 in which R₁, R₂, R₄, R₅ may be the same or different from H, OH, NH₂, NHR, NR₂, OR, R, wherein R represents an alkyl group bearing 1 to 6 carbon atoms in a straight or ramified chain eventually substituted by a group such as OH, OR', NH₂, NHR', NR'₂ wherein R' represents a straight, ramified, or cyclic alkyl chain bearing 1 to 6 carbon atoms, eventually forming an heterocyclic ring by including the heteroatom O or N, and R₃ represents a -CH₂OH or -CH₂OR substituent wherein R is defined as above, wherein
1) a 3-protected hydroxy naphthalene 2-carboxylic acid of formula (IIa), 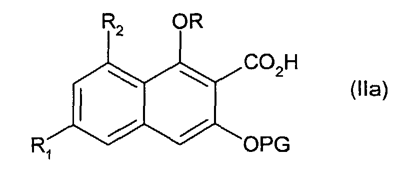 wherein R₁, R₂, and R have the same meaning as for formula (Ia) and PG is a protective group, is condensed with a 4,5,7-trisubstituted 3-halogeno naphthalene of formula (IIb) 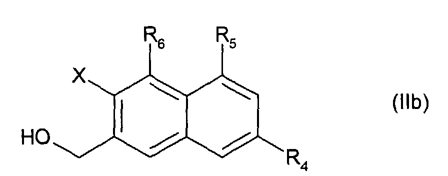 wherein X = halogen, R₄-R₅ have the meaning as in formula (Ia), and R₆ is an alkoxy group bearing 1 to 6 carbon atoms to give a product of formula (II), 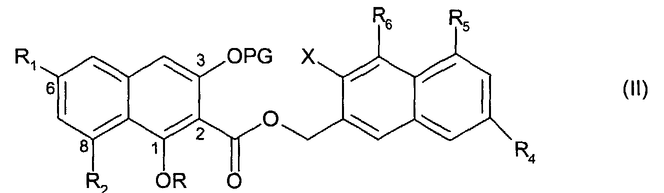 in which X, PG, R, R₁-R₆ have the same meaning as in formula (IIa) and (IIb)
   the product of formula (II) is reacted with an organoalkali metal derivative to give a product of formula (III) 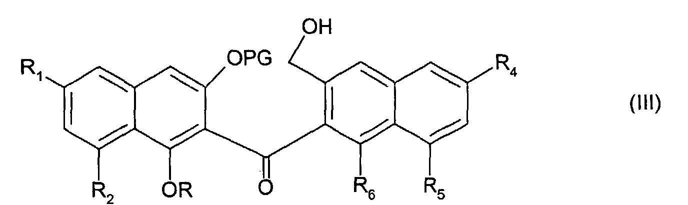
2) the OH of the hydroxymethyl group is protected to give -CH₂OR,
   the OPG group is cleaved to give an OH group,
   the cycle is closed by reaction with a base, thus linking with an oxygen atom, the carbons that bore the OPG and R₆ groups,
   the OH in the α position to the keto group is deprotected to give a product of formula (IVa), 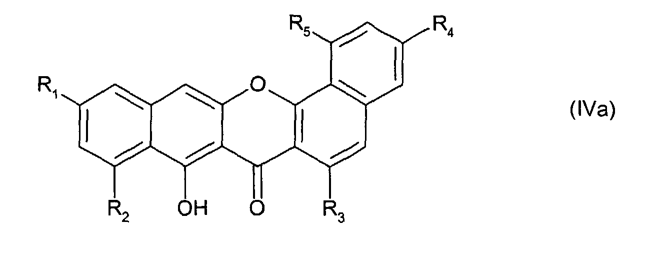 wherein R₁, R₂, R₄ and R₅ have the same meaning as in formula (Ia) and R₃ represents a CH₂OR group, wherein R also has the same meaning as a formula (Ia),
3) the keto group is reduced to give a product of formula (Va) 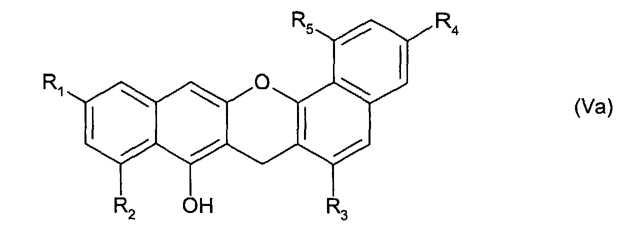 wherein R₁, R₂, R₃, R₄ and R₅ have the same meaning as in formula (IVa)
4) the OH in the α position to the methylene group is protected and the product is then oxidized to give a product of formula (Ia).

In the above process, in the first step the condensation is preferably carried out in the presence of diethyl azodicarboxylate and a phosphine derivative wherein the phosphine derivative is preferably a triarylphosphine.

In the process, in step 1 the metallation is preferably carried out in presence of an alkyllithium preferably butyllithium.

In the process, in step 2 the protection of the hydroxymethyl group is preferably carried out with a methylating agent in presence of a basic compound such as silver oxyde and iodomethane, sodium hydride and iodomethane, or methyl triflate or a salt of trimethyloxonium and a pyridine derivative.

In the process, in step 2 the product obtained is deprotected (OPG) preferably by hydrogenation with palladium hydroxide or palladium on carbon or by treatment with ammonium formate in the presence of palladium on carbon.

In the process, in step 2 the cycle is preferably closed by action of an alkaline base.

In the process, in step 2 the product is preferably deprotected at the OH group α to the keto group by action of lithium chloride, boron trichloride, or zinc and sodium hydroxide.

In the process, in step 3 the keto group is reduced preferably with hydride most preferably in presence of borane-dimethyl sulfide complex.

In the process, in step 4 the product is preferably protected by a silyl group, and the product is finally oxidized by action of a palladium derivative.

Otherwise in the process, in step 4 without any protection the product is oxidized with dichlorodicyanobenzoquinone or silver oxide. Preferably the sequence silylation and oxidation with palladium is used.

The second object of the present invention is related to another process of manufacture of hypoxyxylerone derivatives of formula (Ib) 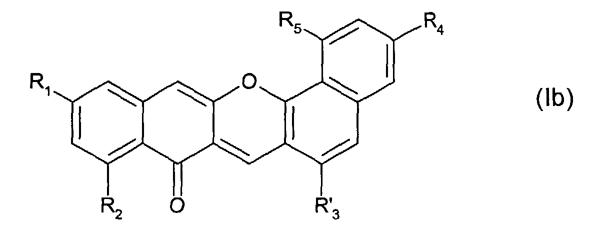 in which R₁, R₂, R₄ and R₅ have the same meaning as in formula (Ia) and R'₃ represents a hydrogen or an R substituent, wherein R has the same meaning as above.
1) a 2-alkoxycarbonylmethyl benzoic acid ester or a derivative of it of formula (VI) 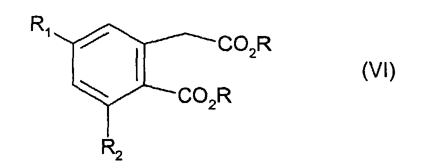 is condensed with a 1-(1-hydroxy, 1-hydroxy-3-alkyl or 1-hydroxy-3-alkoxymethyl naphthalene-2 yl) ethanone or a derivative of it of formula (VII) 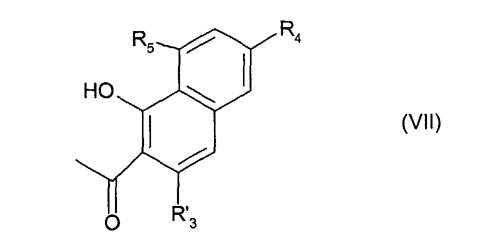 wherein R'₃, R₄ and R₅ have the same meaning as in formula (Ib) in presence of a base to obtain a product of formula (IVb), 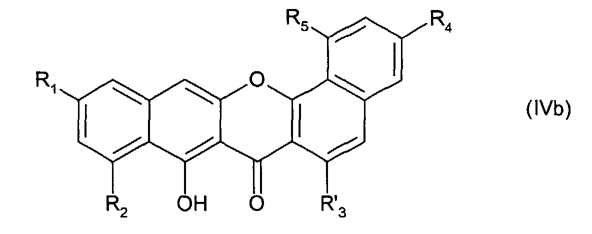 wherein R₁, R₂, R'₃, R₄ and R₅ have the same meaning as in formula (Ib),
2) the keto group is reduced
3) the OH in the α position to the methylene group is protected and the product is then oxidized to give a product of formula (Ib).

Alternatively
2') the hydroxyl protecting group(s) is(are) cleaved
3') silylation is performed
4') the keto group is reduced
5') the silyl group(s) is(are) hydrolysed
6') the product is oxidized to give a product of formula (Ib).
The condensation of compound (VI) with compound (VII) is the key inventive step of this process. In the process, step 1, the base is preferably chosen from hydride and more preferably from sodium or potassium hydride.
In the process, in step 2 the product of formula (IVb) is preferably reduced in the presence of borane-dimethyl sulfide complex.
In the process, in step 3, the product is preferably protected by a silyl group and the product is oxidized in step 3 preferably, with dichlorodicyanobenzoquinone, silver oxyde or palladium derivative.
In the process, in step 2', the product of formula (IVb) is preferably treated with boron tribromide to cleave the hydroxylprotective groups.
In the process, in step 3', the product is preferably silylated with triethylsilyl trifluoromethanesulfonate.
In the process, in step 4', the product is preferably reduced in the presence of borane-dimethyl sulfide complex.
In the process, in step 5', the product is preferably hydrolysed with aqueous hydrochloric acid in methanol.
In the process, in step 6', the product is preferably oxidized by air.

The present invention has as a third object new products of formula (Ib) 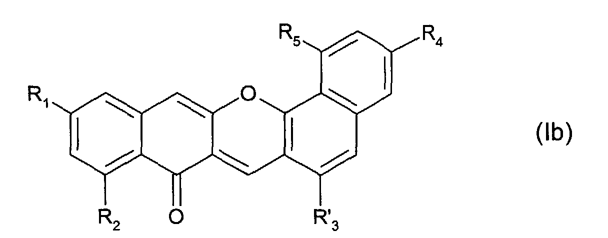 in which R₁, R₂, R₄, R₅ which may be the same or different from H, OH, NH₂, NHR, NR₂, OR, R wherein R represents an alkyl group bearing 1 to 6 carbon atoms in a straight or ramified chain eventually substituted by a group such as OH, OR', NH₂, NHR', NR'₂ wherein R' represents a straight, ramified, or cyclic alkyl chain bearing 1 to 6 carbon atoms, eventually forming an heterocyclic ring by including the heteroatom O or N, and R'₃ represents a hydrogen or an R substituent, wherein R has the same meaning as above, with a disclaimer concerning hypoxyxylerone itself.

The present invention has as a fourth object new products of formula (IVa) and of formula (IVb) which can be represented by a general formula (IV), 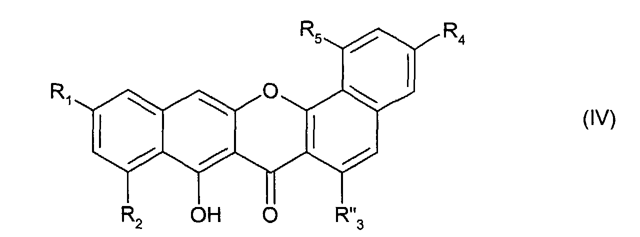 in which R₁, R₂, R₄, R₅ have the same meaning as above in formula (Ia) or (Ib) and wherein R"₃ represents a -CH₂OH or -CH₂OR substituent or a hydrogen or an R substituent.

The present invention has as a fifth object new products of formula (V), 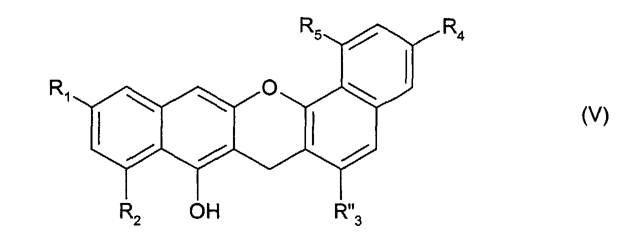 in which R₁, R₂, R₄, R₅ have the same meaning as in formula (Ia) or (Ib) and wherein R"₃ represents a -CH₂OH or -CH₂OR substituent or an hydrogen or an R substituent.

The present invention has a sixth object use of products of formula (Ia) with the inclusion of hydroxyxylerone itself or products of formula (Ib) wherein in R₁, R₂, R₄, R₅ at least one represents OH as medicine.

The present invention has a seventh object use of product of formula (IVb) wherein in R₁, R₂, R₄, R₅ at least one represents OH and R'₃ is R as medicine.

The present invention has a preferred object where the use as medicine is for preparing a medicine for treating cancer.

### Example 1

### - Synthesis of products of formulas (IVa) and (Va) wherein R₁ = R₂ = R₄ = R₅ = OMe and R₃ = CH₂OMe

### - Synthesis of a product of formula (Ia) wherein R₁ = R₂ = R₄ = R₅ = OMe and R₃ = CH₂OMe.

### ● Synthesis of naphthalene alcohol (IIb)

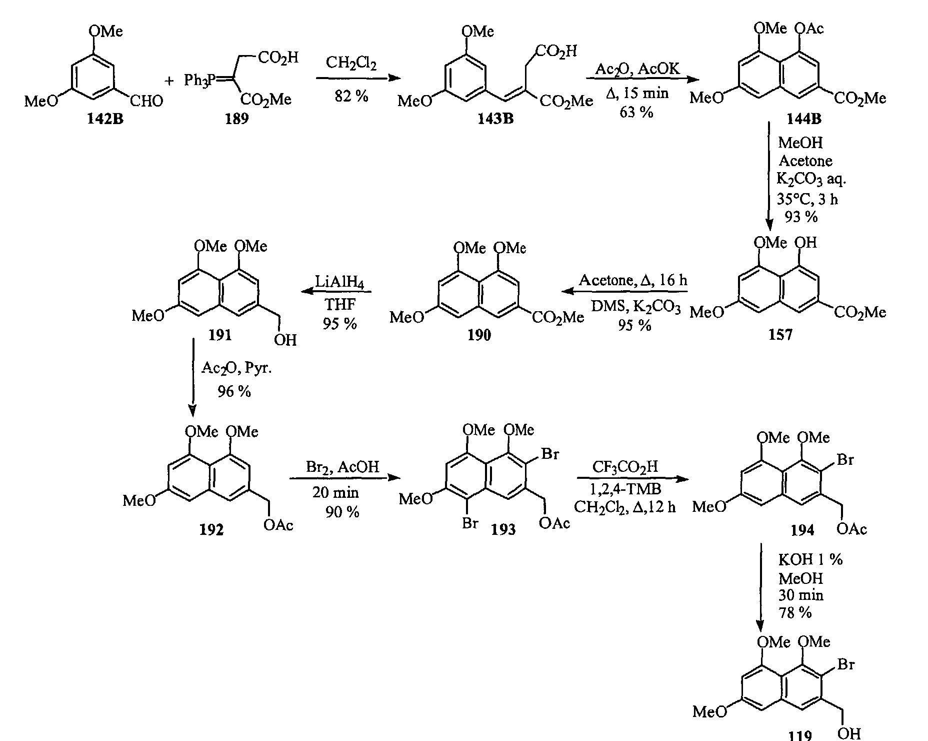

Product 119 is one of the products of general formula (IIb).

### ● Synthesis of naphthalene acid (IIa)

Product 118 is one of the products of general formula (IIa)

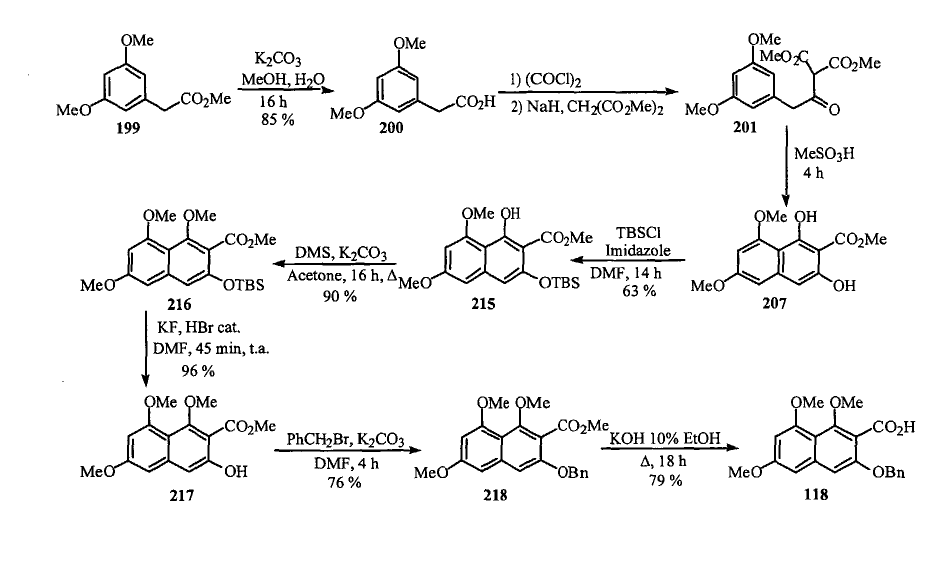

### ● Condensation of (IIa) and (IIb) to give products of

1) **formulas (IVa) and (Va) wherein R**_{**1**} **= R**_{**2**} **= R**_{**4**} **= R**_{**5**} **= OMe and R**_{**3**} **= CH**_{**2**}**OMe**
2) **formula (Ia) wherein R**_{**1**} **= R**_{**2**} **= R**_{**4**} **= R**_{**5**} **= OMe and R**_{**3**} **= CH**_{**2**}**OMe** 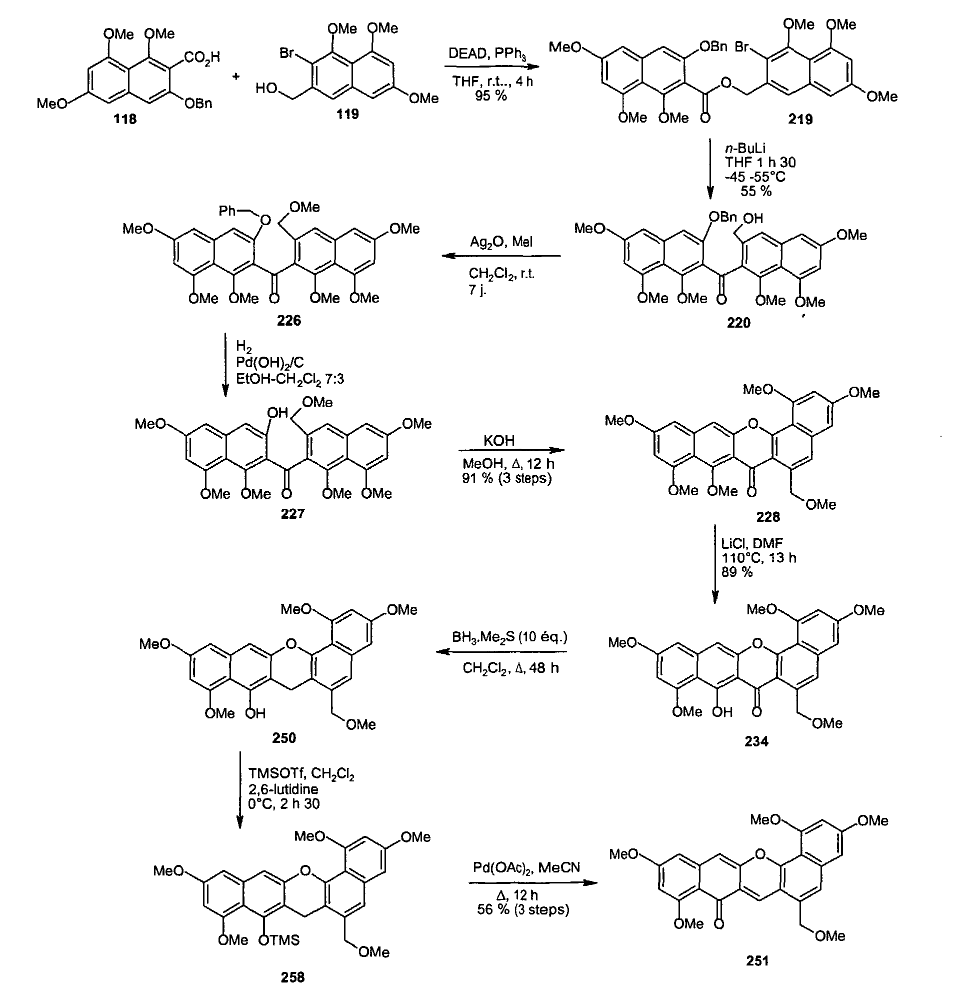 Product 234 is one of the products of general formula (IVa).
   Product 250 is one of the products of general formula (Va).
   Product 251 is one of the products of general formula (Ia).

### ● Synthesis of (IIb)

### 4,5,7-Trimethoxy-naphthalene-2-carboxylic Acid Methyl Ester (190).

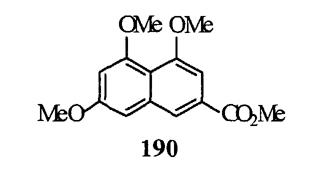

A mixture of naphthol **157** (J. Liu, Z. Diwu, J.W. Lown, Synthesis, 1995, 914-918), (500 mg, 1.91 mmol) and dry potassium carbonate (422 mg, 3.05 mmol) in anhydrous acetone (22 mL) and dimethyl sulfate (289 µL, 3.05 mmol) was stirred under argon at 60°C for 16 hours. After being allowed to cool to room temperature, the mixture was filtered to remove the excess potassium carbonate, which was washed abundantly with acetone. The filtrate was evaporated and the residue was dissolved in dichloromethane, which was washed several times with aqueous NaOH (2 M) and water. After the usual workup, a yellow solid was obtained (500 mg, 95 %).
mp 145°C;
IR (KBr) 2957, 1719, 1624, 1605, 1591 cm⁻¹;
¹H NMR (CDCl₃, 200 MHz) δ 8.00 (d, *J=* 1.7 Hz, 1H), 7.24 (d, *J=* 1.7 Hz, 1H), 6.77 (d, *J=* 2.4 Hz, 1H), 6.57 (d, *J=* 2.4 Hz, 1H), 3.98 (s, 3H), 3.93 (s, 3H), 3.92 (s, 3H), 3.87 (s, 3H);
¹³C NMR (50.3 MHz, CDCl₃) δ 167.1 (C), 158.5 (C), 158.1 (C), 157.4 (C), 137.2 (C), 128.2 (C), 122.7 (CH), 115.1 (C), 102.8 (CH), 100.8 (CH), 99.9 (CH), 56.2 (2CH₃), 55.2 (CH₃), 52.1 (CH₃);
MS (EI) m/z 276 (M⁺, 100);
Anal. calcd for C₁₅H₁₆O₅: C, 65.21; H, 5.84. Found: C, 65.23; H, 5.75.

### (4,5,7-Trimethoxy-naphthalen-2-yl)-methanol (191).

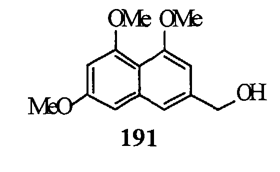

Ester **190** (100 mg, 0.362 mmol) in anhydrous THF (5.8 mL) was added dropwise over 30 minutes to a stirred suspension of lithium aluminium hydride (41 mg, 1.08 mmol) in anhydrous THF (3.7 mL) at ambient temperature. Stirring was continued for 30 minutes and then the reaction was quenched by addition of saturated aqueous NH₄Cl and filtered. The filtrate was diluted with ether and, after the usual workup, alcohol **191** (85 mg, 95 %) was obtained as a white solid.
mp 116°C (hexane-EtOAc);
IR (KBr) 3300, 2931, 2840, 1629, 1612, 1597, 1584 cm⁻¹;
¹H NMR (CDCl₃, 200 MHz) δ 7.18 (d, *J=* 1.2 Hz, 1H), 6.67 (d, *J=* 1.2 Hz, 1H), 6.63 (d, *J=* 2.4 Hz, 1H), 6.46 (d, *J=* 2.4 Hz, 1H), 4.73 (s, 2H), 3.93 (s, 3H), 3.91 (s, 3H), 3.86 (s, 3H);
¹³C NMR (CDCl₃, 50.3 MHz) δ 157.9 (C), 157.8 (C), 157.0 (C), 139.7 (C), 137.7 (C), 117.1 (CH), 112.0 (C), 102.7 (CH), 98.7 (CH), 98.2 (CH), 64.8 (CH₂), 55.8 (2CH₃), 54.8 (CH₃);
MS (CI) m/z 249 (MH⁺, 100), 231 (6);
HRMS calcd for C₁₄H₁₆O₄: 248.1049. Found: 248.1072 (M⁺).

### Acetic Acid 4,5,7-Trimethoxy-naphthalen-2-ylmethyl Ester (192).

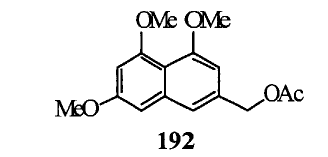

Alcohol **191** (75 mg, 0.302 mmol) was dissolved in acetic anhydride (58 µL, 1.01 mmol) and pyridine (540 µL) and then heated at 50°C for 90 minutes. After being allowed to cool to room temperature, the mixture was poured into water and extracted with ether. The organic layer was successively washed with saturated aqueous NaHCO₃, aqueous HCl (10 %), water, saturated aqueous CuSO₄, and brine, dried over anhydrous Na₂SO₄, and concentrated under reduced pressure. Acetate **192** (84 mg, 96 %) was obtained as a white-yellow solid.
mp 92°C (hexane-EtOAc);
IR (KBr) 3074, 2937, 2846, 1737, 1620 cm⁻¹;
¹H NMR (CDCl₃, 200 MHz) δ 7.22 (s, 1H), 6.65 (d, *J*= 2.4 Hz, 1H), 6.63 (d, *J=* 1.4 Hz, 1H), 6.47 (d, *J=* 2.4 Hz, 1H), 5.14 (s, 2H), 3.92 (s, 3H), 3.89 (s, 3H), 3.84 (s, 3H), 2.11 (s, 3H);
¹³C NMR (CDCl₃, 50.3 MHz) δ 170.8 (C), 158.3 (C), 158.1 (C), 157.5 (C), 137.9 (C), 134.5 (C), 119.1 (CH), 112.6 (C), 103.6 (CH), 98.9 (CH), 98.8 (CH), 66.3 (CH₂), 56.1 (2CH₃), 55.1 (CH₃), 20.9 (CH₃);
MS (CI, NH₃+isobutane) m/z 291 (MH⁺, 100), 248 (10), 231 (75);
Anal. calcd for C₁₆H₁₈O₅: C, 66.20; H, 6.25. Found: C, 66.44; H, 6.43.

### Acetic Acid 3,8-Dibromo-4,5,7-trimethoxy-naphthalen-2-ylmethyl Ester (193).

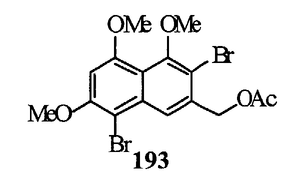

A solution of bromine (98 µL, 19.1 mmol) in acetic acid (37 mL) was added dropwise to a solution of acetate **192** (2.41 g, 8.32 mmol) and anhydrous sodium acetate (1.71 g, 20.8 mmol) in acetic acid (37 mL). The mixture was stirred for 20 minutes and then filtered. The filtered material was washed several times with pentane and dried to furnish **193** (3.35 g, 90 %) as a yellow solid.
mp 183°C (hexane-EtOAc);
IR (KBr) 3000, 2945, 2840, 1743, 1606, 1588 cm⁻¹;
¹H NMR (CDCl₃, 200 MHz) δ 8.03 (s, 1H), 6.67 (s, 1H), 5.29 (s, 2H), 4.00 (s, 6H), 3.84 (s, 3H), 2.19 (s, 3H);
¹³C NMR (CDCl₃, 50.3 MHz) δ 170.5 (C), 156.5 (C), 154.6 (C), 153.7 (C), 135.4 (C), 133.9 (C), 122.5 (CH), 116.9 (C), 114.1 (C), 99.9 (C), 96.0 (CH), 66.1 (CH₂), 61.6 (CH₃), 56.9 (CH₃), 56.6 (CH₃), 20.9 (CH₃);
MS (DCI, NH₃+isobutane) m/z 449 (MH⁺, 97), 406 (56), 389 (100), 369 (38);
Anal. calcd for C₁₆H₁₆Br₂O₅: C, 42.89; H, 3.60. Found: C, 42.66; H, 3.71.

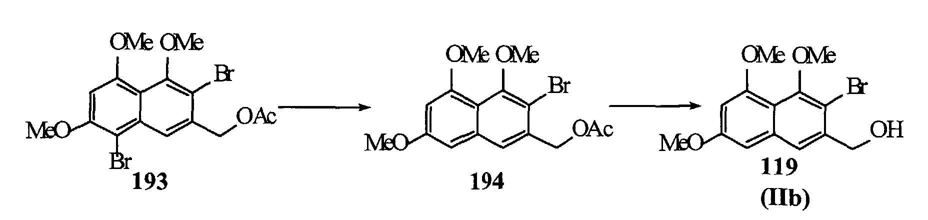

### Acetic Acid 3-Bromo-4,5,7-trimethoxy-naphthalen-2-ylmethyl Ester (194).

A solution of naphthalene **193** (1.01 g, 2.25 mmol), 1,2,4-trimethoxybenzene (502 µL, 3.88 mmol) and trifluoroacetic acid (1.73 mL, 22.5 mmol) in dichloromethane (26.5 mL) was stirred at 40°C for 12 hours. After being allowed to cool to room temperature, the reaction was quenched by addition of saturated aqueous NaHCO₃ and extracted with dichloromethane. After the usual workup, the crude product (1.51 g) was obtained, which was used without further purification.
¹H NMR (CDCl₃, 200 MHz) δ 7.45 (s, 1H), 6.67 (d, *J=* 2.4 Hz, 1H), 6.51 (m, 1H), 5.24 (s, 2H), 3.95-3.85 (m, 9H), 2.15 (s, 3H).

### (3-Bromo-4,5,7-trimethoxy-naphthalen-2-yl)-methanol 119 (IIb).

The crude product **194** (1.51 g) dissolved in a 1 % (w/v) solution of potassium hydroxide (189 mg, 3.37 mmol) in methanol was stirred at ambient temperature for 30 minutes. The reaction was quenched by addition of HCl (0.1 N) and then concentrated under reduced pressure. The residue was dissolved in ether and, after the usual workup, the crude product was purified by flash column chromatography (silica gel treated with triethylamine 2.5 % w/v, cyclohexane-EtOAc 7:3) to afford the alcohol 119 **IIb** (573 mg, 78 % for two steps).
mp 104-105°C (hexane-EtOAc) white needles;
IR (neat) 3480, 2934, 2837, 1619, 1599, 1581 cm⁻¹;
¹H NMR (CDCl₃, 200 MHz) δ 7.51 (s, 1H), 6.67 (d, *J*= 2.1 Hz, 1H), 6.51 (d, *J=* 2.1 Hz, 1H), 4.80 (d, *J=* 6.2 Hz, 2H), 3.95 (s, 3H), 3.86 (s, 3H), 3.85 (s, 3H), 2.26 (t, *J=* 6.5 Hz, 1H);
¹³C NMR (CDCl₃, 50.3 MHz) δ 158.3 (C), 156.4 (C), 153.0 (C), 138.7 (C), 136.6 (C), 121.9 (CH), 115.8 (C), 112.6 (C), 99.3 (CH), 98.8 (CH), 65.1 (CH₂), 61.5 (CH₃), 56.0 (CH₃), 55.2 (CH₃);
MS (EI) 328 (M⁺, 50), 31 (100);
Anal. calcd for C₁₄H₁₅BrO₄: C, 51.40; H, 4.62. Found: C, 51.27; H, 4.54.

### ● Synthesis of IIa

### 2-[2-(3,5-Dimethoxy-phenyl)-ethanoyl]-malonic Acid Dimethyl Ester (201).

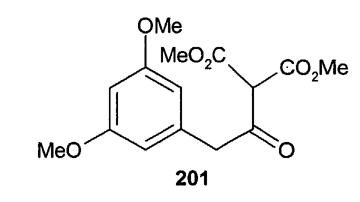

To a stirred solution of (3,5-dimethoxyphenyl) acetic acid (2.5 g, 12.9 mmol) in anhydrous toluene (25 mL) and DMF (1 drop) under argon at 0°C was added dropwise oxalyl chloride (4.5 mL, 51.6 mmol). After being stirred at room temperature for 2 hours, the solution was concentrated under reduced pressure and the resulting crude (3,5-dimethoxyphenyl) acetyl chloride was immediately used.

To a mixture of sodium hydride (60 % in mineral oil) (2.83 g, 70.8 mmol) in anhydrous THF (250 mL) under argon at 0°C was added dropwise dimethyl malonate (8.4 mL, 73.4 mmol). The mixture was stirred at ambient temperature until the solution became clear, then cooled in an ice bath, and treated dropwise with a solution of the acid chloride in anhydrous THF (25 mL). The resulting solution was stirred at 60°C for 20 hours and then hydrolysed by addition of aqueous HCl (1 M). The aqueous layer was extracted with ether. After the usual workup of the organic layer, a yellow oil was obtained (11.9 g), which was chromatographed (silica gel, hexane-EtOAc 7:3). The excess dimethyl malonate was removed by distillation under reduced pressure to leave a white solid **201** (3.00 g, 75 %).

The complexity of the NMR spectrum described below is due to the equilibrium of compound **201** and its enolic form.
mp 77°C (hexane-EtOAc);
IR (neat) 3019, 2952, 2895, 2838, 1726, 1597 cm⁻¹;
¹H NMR (CDCl₃, 200 MHz) δ 6.50-6.30 (m,3H), 3.80-3.65 (m, 15H);
¹³C NMR (CDCl₃, 50.3 MHz) δ 196.3 (C), 180.4 (C), 171.4 (C), 166.2 (C), 164.8 (C), 161.0 (C), 160.8 (C), 137.3 (C), 134.6 (C), 107.6 (CH), 107.2 (CH), 99.8 (C), 99.5 (CH), 99.1 (CH), 63.5 (CH), 55.2 (CH₃), 53.1 (CH₃), 52.4 (CH₃), 52.2 (CH₃), 49.1 (CH₂), 39.8 (CH₂);
MS (CI) m/z 311 (MH⁺, 9.3), 197 (100) ;
Anal. calcd for C₁₅H₁₈O₇: C, 58.06; H, 5.85. Found: C, 57.75; H, 5.85.

### 1,3-Dihydroxy-6,8-dimethoxy-naphthalene-2-carboxylic Acid Methyl Ester (207).

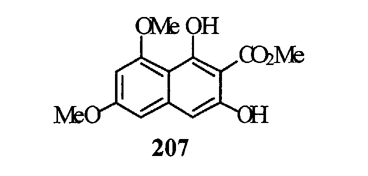

Diester **201** (5.12 g, 16.5 mmol) was stirred in methanesulfonic acid (5 mL) for 4 hours at room temperature. The reaction mixture was then poured into cold water and the aqueous layer was extracted with CH₂Cl₂. After the usual workup of the organic layer, naphthalene **207** (3.98 g, 90 %) was obtained, which was used without further purification.
¹H NMR (CDCl₃, 200 MHz) δ 11.10 (s, 1H), 10.67 (s, 1H), 6.60 (s, 1H), 6.45 (d, *J=* 2.1 Hz, 1H), 6.25 (d, *J=* 2.1 Hz, 1H), 4.01 (s, 3H), 3.98 (s, 3H), 3.86 (s, 3H).

### 3-(tert-Butyl-dimethyl-silanyloxy)-1-hydroxy-6,8-dimethoxy-naphthalene-2-carboxylic Acid Methyl Ester (215).

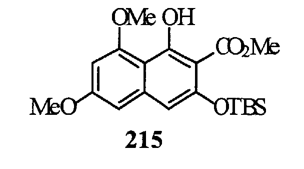

Under argon, a solution of crude naphthalene **207** (3.98 g, 13.3 mmol), *tert*butyldimethylsilyl chloride (5.4 g, 35.8 mmol), and imidazole (2.45 g, 35.8 mmol) in anhydrous DMF (130 mL) was stirred at ambient temperature for 14 hours and then diluted with ether. The organic layer was successively washed with saturated aqueous NaHCO₃, water, and brine, dried over anhydrous Na₂SO₄, filtered, and concentrated in vacuo. The crude product was purified by silica gel chromatography (hexane-EtOAc 9:1, 8:2, and then 6:4) to afford **215** as a yellow oil (4.09 g, 63 % for two steps).
IR (neat) 3380, 2957, 2929, 2859, 1735, 1639 cm⁻¹;
¹H NMR (CDCl₃, 200 MHz) δ 10.15 (s, 1H), 6.50 (s, 1H), 6.47 (d, *J=* 2 Hz, 1H), 6.27 (d, *J=* 2 Hz, 1H), 3.91 (s, 3H), 3.88 (s, 3H), 3.83 (s, 3H), 0.97 (s, 9H), 0.24 (s, 6H);
¹³C NMR (CDCl₃, 50.3 MHz) δ 167.6 (C), 159.1 (C), 157.7 (C), 155.0 (C), 151.9 (C), 138.3 (C), 108.6 (C), 105.9 (C), 105.1 (CH), 98.1 (CH), 96.1 (CH), 55.8 (CH₃), 55.0 (CH₃), 51.8 (CH₃), 25.3 (CH₃), 17.9 (C), -4.6 (CH₃);
MS (CI, NH₃+isobutane) m/z 393 (MH⁺, 100), 361 (87);
Anal. calcd for C₂₀H₂₈O₆Si: C, 61.20; H, 7.19. Found: C, 61.02; H, 7.40.

### 3-(tert-Butyl-dimethyl-silanyloxy)-1,6,8-trimethoxy-naphthalene-2-carboxylic Acid Methyl Ester (216).

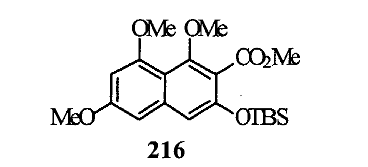

A mixture of naphthalene **215** (1.48 g, 3.77 mmol) and dry potassium carbonate (835 mg, 6.04 mmol) in anhydrous acetone (45 mL) and dimethyl sulfate (0.57 mL, 6.02 mmol) was refluxed for 16 hours. After being allowed to cool to room temperature, the reaction mixture was filtered to remove the excess potassium carbonate, which was washed abundantly with acetone. The filtrate was concentrated in vacuo and the residue was dissolved in ether. The organic layer was washed several times with aqueous NaOH (2 N) and, after the usual workup, the resultant crude product was purified by silica gel chromatography (hexane-EtOAc 7:3) to afford **216** (1.38 g, 90 %) as colorless crystals.
mp 91°C (hexane-EtOAc);
IR (neat) 2960, 2938, 2858, 1740, 1621, 1580 cm⁻¹;
¹H NMR (CDCl₃, 200 MHz) δ 6.78 (s, 1H), 6.54 ( d, *J* = 2.2 Hz, 1H), 6.37 (d, *J*= 2.2 Hz, 1H), 3.92 (s, 3H), 3.87 (s, 6H), 3.84 (s, 3H), 0.97 (s, 9H), 0.26 (s, 6H);
¹³C NMR (CDCl₃, 50.3 MHz) δ 167.0 (C), 159.1 (C), 157.6 (C), 155.3 (C), 150.8 (C), 138.8 (C), 119.9 (C), 111.0 (C), 109.9 (CH), 97.9 (CH), 97.4 (CH), 63.7 (CH₃), 55.8 (CH₃), 55.2 (CH₃), 52.0 (CH₃), 25.4 (CH₃), 18.0 (C), -4.4 (CH₃);
MS (CI, NH₃+isobutane) m/z 407 (MH⁺, 70), 375 (100);
Anal. calcd for C₂₁H₃₀O₆Si: C, 62.04; H, 7.44. Found: C, 62.10; H, 7.39.

### 3-Hydroxy-1,6,8-trimethoxy-naphthalene-2-carboxylic Acid Methyl Ester (217).

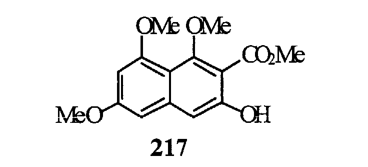

A solution of naphthalene **216** (2.25 g, 5.54 mmol), potassium fluoride (641 mg, 11.1 mmol), and hydrobromic acid (48 % in water, 63 µL, 0.557 mmol) in DMF (38 mL) was stirred at ambient temperature for 45 minutes. This solution was then poured into a mixture of dilute HCl (0.1N) and ether, and the aqueous layer was extracted with ether. After the usual workup, the crude product was chromatographed (silica gel, hexane-EtOAc 7:3) to produce a yellow cotton-like solid (1.55 g, 96 %).
mp 107°C (hexane-EtOAc);
IR (KBr) 3229, 2976, 2935, 2843, 1663, 1625, 1608 cm⁻¹;
¹H NMR (CDCl₃, 200 MHz) δ 10.70 (s, 1H), 6.89 (s, 1H), 6.45 (d, *J*= 2.1 Hz, 1H), 6.27 (d, *J*= 2.1 Hz, 1H), 3.98 (s, 3H), 3.90 (s, 3H), 3.83 (s, 3H), 3.81 (s, 3H);
¹³C NMR (CDCl₃, 50.3 MHz) δ 170.8 (C), 161.2 (C), 160.6 (C), 158.6 (C), 157.0 (C), 141.3 (C), 110.6 (C), 106.9 (CH), 106.2 (C), 96.9 (CH), 96.8 (CH), 63.2 (CH₃), 55.6 (CH₃), 54.9 (CH₃), 52.3 (CH₃);
MS (DCI) m/z 293 (MH⁺, 100), 261 (45); (EI) m/z 292 (M⁺, 25), 260 (100), 217 (79);
Anal. calcd for C₁₅H₁₆O₆: C, 61.64; H, 5.52. Found: C, 61.92; H, 5.59.

### 3-Benzyloxy-1,6,8-trimethoxy-naphthalene-2-carboxylic Acid Methyl Ester (218).

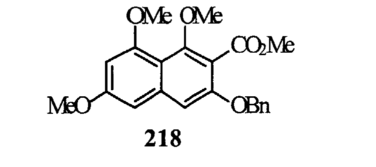

A solution of naphthalene **217** (491 mg, 1.68 mmol), dry potassium carbonate (348 mg, 2.52 mmol) and benzyl bromide (240 µL, 2.02 mmol) in anhydrous DMF (12 mL) was stirred at room temperature under argon for 4 hours and then poured into a mixture of dilute HCl (0.1 N) and ether, and the aqueous layer was extracted with ether. After the usual workup, the crude product was purified by silica gel chromatography (hexane-EtOAc 9:1, 8:2 and then 7:3) to afford a white solid **218** (490 mg, 76 %).
mp 111 °C (pentane-ether);
IR (neat) 2933, 2845, 1730, 1620 cm⁻¹;
¹H NMR (CDCl₃, 200 MHz) δ 7.45-7.25 (m, 5H), 6.86 (s, 1H), 6.59 (d, *J=* 2.1 Hz, 1H), 6.38 (d, *J=* 2.1 Hz, 1H), 5.15 (s, 2H), 3.92 (s, 3H), 3.91 (s, 3H), 3.88 (s, 3H), 3.84 (s, 3H);
¹³C NMR (CDCl₃, 50.3 MHz) δ 167.0 (C), 159.3 (C), 157.6 (C), 153.8 (C), 139.0 (C), 136.5 (C), 128.4 (CH), 127.7 (CH), 126.8 (CH), 117.3 (C), 110.7 (C), 103.4 (CH), 98.4 (CH), 97.3 (CH), 70.0 (CH₂), 63.8 (CH₃), 55.9 (CH₃), 55.2 (CH₃), 52.3 (CH₃);
MS (CI, NH₃+isobutane) m/z 383 (MH⁺, 100), 351 (12);
Anal. calcd for C₁₅H₁₆O₆: C, 61.64; H, 5.52. Found: C, 61.92; H, 5.59.

### 3-Benzyloxy-1,6,8-trimethoxy-naphthalene-2-carboxylic Acid 118 (IIa).

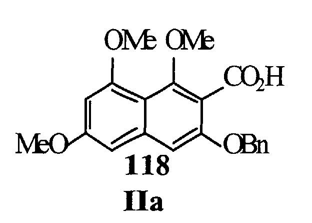

Naphthalene **218** (80 mg, 0.21 mmol) was refluxed in a 10 % solution of KOH in ethanol for 18 hours. After being allowed to cool to room temperature, the mixture was concentrated in vacuo, and the resulant residue was dissolved in aqueous NaOH (3.5 N), and then washed with ether. The aqueous layer was acidified to pH 1 with aqueous HCl (6 N) and then extracted with dichlorométhane. After the usual workup, a white solid was obtained (61 mg, 79 %).
mp 191°C (hexane-EtOAc);
IR (neat) 3008, 2929, 2850, 1696, 1621 cm⁻¹;
¹H NMR (CDCl₃, 200 MHz) δ 7.50-7.20 (m, 5H), 6.72 (s, 1H), 6.51 (d, *J=* 2.1 Hz, 1H), 6.33 (d, *J=* 2.1 Hz, 1H), 5.13 (s, 2H), 3.89 (s, 3H), 3.87 (s, 6H);
¹³C NMR (CDCl₃, 50.3 MHz) δ 168.7 (C), 159.6 (C), 157.4 (C), 155.0 (C), 153.5 (C), 139.1 (C), 136.3 (C), 128.6 (CH), 126.9 (CH), 116.3 (C), 110.3 (C), 103.6 (CH), 98.3 (CH), 97.2 (CH), 70.1 (CH₂), 63.9 (CH₃), 55.8 (CH₃), 55.3 (CH₃);
MS (EI) m/z 368 (M⁺, 13), 91 (100);
Anal. calcd for C₂₁H₂₀O₆: C, 68.47; H, 5.47. Found: C, 68.23; H, 5.58.

### ● Synthesis of xanthone IVa(R₁ = R₂ = R₄ = R₅ = OMe and R₃ = CH₂OMe)

### 3-Benzyloxy-1,6,8-trimethoxy-naphthalene-2-carboxylic Acid 3-Bromo-4,5,7-trimethoxy-naphthalen-2-ylmethyl Ester (219).

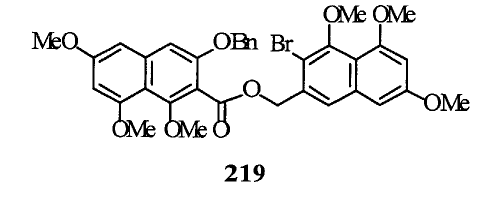

To a stirred solution of acid **118** (203 mg, 0.552 mmol), alcohol **119** (180 mg, 0.549 mmol), and triphenylphosphine (159 mg, 0.606 mmol) in anhydrous THF (6.8 mL) at 0°C under argon was added dropwise diethyl azodicarboxylate (96 µL, 0.610 mmol). Stirring was continued at room temperature for 3 hours, whereupon the mixture was diluted with ether and, after the usual workup, the crude product was chromatographed (silica gel, hexane-EtOAc 7:3). The residue obtained was dissolved in ether and washed several times with aqueous HCl (6 N) (to remove the byproduct of reduced DEAD) and brine, dried over anhydrous Na₂SO₄, filtered, and concentrated under reduced pressure to afford ester **219** as a white solid (356 mg, 95 %).
mp 136-138°C (hexane-EtOAc);
IR (neat) 2926, 2850, 1728, 1619, 1580 cm⁻¹;
¹H NMR (CDCl₃, 200 MHz) δ 7.66 (s, 1H), 7.40-7.10 (m, 5H), 6.86 (s, 1H), 6.59 (d, *J=* 2.1 Hz, 2H), 6.50 (d, *J=* 2.4 Hz, 1H), 6.39 (d, *J=* 2.1 Hz, 1H), 5.58 (s, 2H), 5.20 (s, 2H), 3.94 (s, 3H), 3.93 (s, 3H), 3.88 (s, 3H), 3.85 (s, 3H), 3.84 (s, 3H), 3.77 (s, 3H);
¹³C NMR (CDCl₃, 50.3 MHz) δ 166.1 (C), 159.3 (C), 158.4 (C), 157.5 (C), 156.4 (C), 155.4 (C), 153.8 (C), 153.4 (C), 139.1 (C), 136.5 (C), 134.1 (C), 128.4 (CH), 126.6 (CH), 123.2 (CH), 117.1 (C), 116.2 (C), 113.0 (C), 110.7 (C), 103.5 (CH), 99.6 (CH), 98.9 (CH), 98.4 (CH), 97.3 (CH), 69.9 (CH₂), 66.7 (CH₂), 63.9 (CH₃), 61.5 (CH₃), 56.1 (CH₃), 55.9 (CH₃), 55.2 (CH₃), 55.1 (CH₃);
MS (DCI) m/z 679 (MH⁺, 100), 369 (8), 351 (41), 311 (77), 233 (14);
HRMS calcd for C₃₅H₃₄BrO₉: 677.1386. Found: 677.1339 (MH⁺).

### 1-(3-Benzyloxy-1,6,8-trimethoxy-naphthalen-2-yl)-1-(3-hydroxymethyl-1,6,8-trimethoxy-naphthalen-2-yl)-methanone (220).

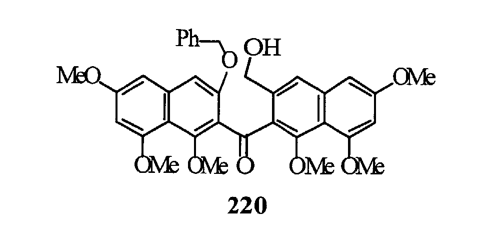

Under argon, ester **219** (269 mg, 0.397 mmol) was dissolved in THF (1.59 mL, C=0.25 M, from which residual water had been removed with *n*-butyllithium and *o*-phenanthroline), and cooled to -45/-55°C. *n*-Butyllithium (203 µL, 0.437 mmol) was then added dropwise. After the solution was stirred for 2 hours, the reaction mixture was quenched by addition of saturated aqueous NH₄Cl, allowed to warm to ambient temperature, and diluted with ethyl acetate. After the usual workup, the crude product was purified by radial thin-layer chromatography (hexane-EtOAc 9:1, 8:2, 7:3, and then 5:5) to give a white solid **220** (130 mg, 55 %), ester **219** (43 mg, 0.064 mmol) and debrominated ester **219A** (32 mg, 0.054 mmol).
mp 220-224°C (cyclohexane-dichloromethane);
IR (neat) 3454, 2957, 2926, 2853, 1619, 1573, 1458 cm⁻¹;
¹H NMR (CDCl₃, 200 MHz) δ 7.41 (s, 1H), 7.35-7.05 (m, 5H), 6.87 (s, 1H), 6.70 (d, *J* =2.1 Hz, 1H), 6.60 (d, *J* = 2.1 Hz, 1H), 6.45 (d, *J* =2.4 Hz, 1H), 6.37 (d, *J =* 2.4 Hz, 1H), 5.03 (s, 2H), 4.55 (d, *J =* 6.8 Hz, 2H), 3.90 (s, 6H), 3.87 (s, 3H), 3.83 (s, 3H), 3.57 (s, 3H), 3.41 (s, 3H);
¹³C NMR (CDCl₃, 75.4 MHz) δ 198.6 (C), 159.6 (C), 159.4 (C), 157.8 (C), 157.6 (C) 156.0 (C), 155.1 (C), 139.5 (C), 139.3 (C), 136.3 (C), 131.3 (C), 128.3 (CH), 127.8 (CH), 127.4 (CH), 125.4 (C), 124.4 (CH), 114.7 (C), 111.1 (C), 103.2 (CH), 99.3 (CH), 98.7 (CH), 97.4 (CH), 70.2 (CH₂), 64.7 (CH₂), 64.1 (CH₃), 64.0 (CH₃), 63.9 (CH₃), 56.0 (CH₃), 55.4 (CH₃), 55.3 (CH₃);
MS (DCI, NH₃+isobutane) m/z 599 (MH⁺, 50), 279 (100);
HRMS calcd for C₃₅H₃₄O₉: 598.2203. Found: 598.2211 (MH⁺).

### 1-(3-Benzyloxy-1,6,8-trimethoxy-naphthalen-2-yl)-1-(1,6,8-trimethoxy-3-methoxy methyl-naphthalen-2-yl)-methanone (226).

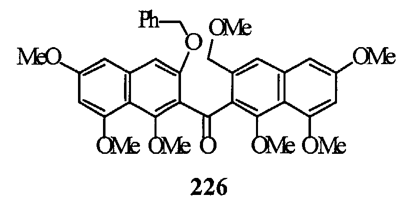

To a solution of alcohol **220** (180 mg, 0.301 mmol) in dichloromethane (3.2 mL) at room temperature was added silver (I) oxide (280 mg, 1.25 mmol) and iodomethane (150 µL, 2.41 mmol). The reaction mixture was stirred in the dark. After 48 hours, 72 hours, 96 hours, and 144 hours, additional silver (I) oxide (145 mg, 0.626 mmol) and iodomethane (70 µL, 1.12 mmol) were added (until disappearance of the starting material in thin-layer chromatography). The slurry was then filtered and the solvent was removed in vacuo to give the product **226** (190 mg).
mp 175-178°C (pentane-dichloromethane, white solid);
IR (neat) 2935, 2845, 1662, 1620, 1578 cm⁻¹;
¹H NMR (CDCl₃, 200 MHz) δ 7.65 (s, 1H), 7.20 (s, 5H), 6.86 (s, 1H), 6.74 (d, *J=* 2.1 Hz, 1H), 6.59 (d, *J=* 2.1 Hz, 1H), 6.43 (d, *J=* 2.1 Hz, 1H), 6.35 (d, *J=* 2.4 Hz, 1H), 5.08 (s, 2H), 4.73 (s, 2H), 3.88 (s, 6H), 3.85 (s, 3H), 3.83 (s, 3H), 3.47 (s, 3H), 3.43 (s, 3H);
¹³C NMR (CDCl₃, 75.4 MHz) δ 196.6 (C), 159.2 (2C), 157.7 (C), 157.6 (C), 156.6 (C), 156.0 (C), 155.1 (C), 139.2 (C), 138.9 (C), 137.1 (C), 136.4 (C), 130.2 (C), 128.3 (CH), 127.6 (CH), 127.1 (CH), 125.3 (C), 120.7 (CH), 114.0 (C), 110.8 (C), 103.3 (CH), 99.1 (CH), 98.7 (CH), 98.5 (CH), 97.1 (CH), 71.8 (CH₂), 70.0 (CH₂), 64.0 (CH₃), 63.6 (CH₃), 58.6 (CH₃), 55.9 (2CH₃), 55.3 (CHₛ). 55.2 (CH₃);
MS (EI) m/z 612 (M⁺, 43), 581 (100), 459 (41);
HRMS calcd for C₃₆H₃₆LiO₉: 619.2519. Found: 619.2536 (MLi⁺).

### 1-(3-Hydroxy-1,6,8-trimethoxy-naphthalen-2-yl)-1-(1,6,8-trimethoxy-3-methoxymethyl-naphthalen-2-yl)-methanone (227).

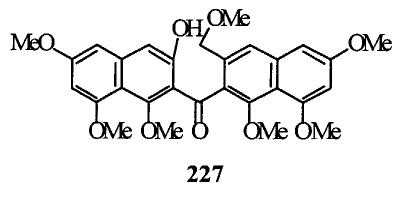

The product **226** (190 mg) and Pd(OH)₂/C (68 mg) were stirred in dichloromethane (5.2 mL) and ethanol (13 mL) under a hydrogen atmosphere for 16 hours. After removal of the catalyst by filtration, the filtrate was concentrated under reduced pressure to give a yellow solid (170 mg).
mp 115-116°C;
IR (KBr) 3452, 2935, 2849, 1640, 1621, 1560 cm⁻¹;
¹H NMR (CDCl₃, 200 MHz) δ 11.97 (s, 1H), 7.47 (s, 1H), 6.92 (s, 1H), 6.74 (d, *J=* 2.1 Hz, 1H), 6.52 (d, *J=* 2.1 Hz, 1H), 6.49 (d, *J=* 2.4 Hz, 1H), 6.19 (d, *J=* 2.1 Hz, 1H), 4.41 (s, 2H), 3.92 (s, 3H), 3.90 (s, 3H), 3.87 (s, 3H), 3.78 (s, 3H), 3.76 (s, 3H), 3.24 (s, 3H), 3.17 (s, 3H);
¹³C NMR (CDCl₃, 75.4 MHz) δ 201.5 (C), 162.8 (2C), 161.3 (C), 158.8 (C), 158.6 (C), 158.3 (C), 157.4 (C), 152.9 (C), 142.6 (C), 137.6 (C), 134.5 (C), 132.1 (C), 121.4 (CH), 115.9 (C), 114.8 (C), 110.1 (C), 107.1 (CH), 99.1 (2CH), 97.4 (CH), 96.7 (CH), 72.4 (CH₂), 64.2 (CH₃), 63.4 (CH₃), 58.4 (CH₃), 56.0 (CH₃), 55.7 (CH₃), 55.3 (CH₃), 55.2 (CH₃);
MS (DCI, NH₃+isobutane) m/z 523(MH⁺, 100), 493 (19), 289 (15), 261 (64), 233 (22);
HRMS calcd for C₂₉H₃₀LiO₉: 529.2050. Found: 529.2071 (MLi⁺).

### 1,3,8,9,11-Pentamethoxy-6-methoxymethyl-14-oxa-benzo[α]naphthacen-7-one (228).

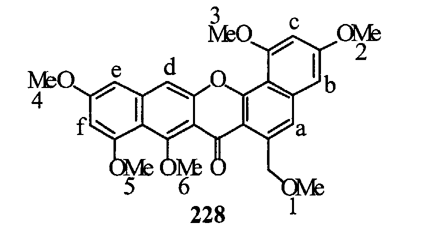

A solution of the product **227** (170 mg) and potassium hydroxide (46 mg, 0.829 mmol) in methanol (10.9 mL) was refluxed overnight. After being allowed to cool to room temperature, the reaction was quenched by addition of dilute aqueous HCl (0.1 N) and the solvents were removed under reduced pressure. The residue was treated with aqueous HCl (0.1 N) and then extracted with dichloromethane. After the usual workup, the resultant crude product was triturated with ether to leave xanthone **228** as a yellow solid (155 mg, 91 % overall yield for three steps).
mp 238-240°C;
IR (KBr) 2936, 2850, 1647, 1617, 1558 cm⁻¹;
UV λmax (CHCl₃)/ nm 242 (log ε, 4.63), 297 (4.83), 412 (4.07);
¹H NMR (CDCl₃, 500 MHz) δ 7.74 (s, 1H, Hₐ), 7.48 (s, 1H, H_{d}), 6.76 (d, *J=* 2.4 Hz, 1H, H_{b}), 6.67 (d, *J =* 2.1 Hz, 1H, Hₑ), 6.56 (d, *J =* 2.4 Hz, 1H, H_{c}), 6.40 (d, *J=* 2.1 Hz, 1H, H_{f}), 5.28 (s, 2H), 4.06 (s, 3H, H₃ ou H₆), 4.04 (s, 3H, H₃ ou H₆), 3.97 (s, 3H, H₅), 3.92 (s, 3H, H₄ ou H₂), 3.91 (s, 3H, H₄ ou H₂), 3.62 (s, 3H, H₁);
¹³C NMR (CDCl₃, 125.7 MHz) δ 178.2 (C), 161.1 (C), 160.6 (C), 160.0 (C), 159.6 (C), 159.4 (C), 155.6 (C), 153.4 (C), 140.3 (C), 139.8 (C), 138.1 (C), 119.1 (CH, Ca), 115.4 (C), 114.0 (2C), 109.5 (C), 108.3 (CH, Cd), 99.6 (CH, Cb ou Cc), 99.3 (CH, Cb ou Cc), 98.5 (CH, Cf), 97.3 (CH, Ce), 73.8 (CH₂), 63.4 (CH₃, C3 ou C6), 59.0 (CH₃, Cl), 56.3 (2CH₃, C5 et C3 ou C6), 55.5 (2CH₃, C2 et C4);
MS (EI) 490 (M⁺, 5), 475 (50), 232 (20);
HRMS calcd for C₂₈H₂₇O₈: 491.1706. Found: 491.1705 (MH⁺).

### 8-Hydroxy-1,3,9,11-tetramethoxy-6-methoxymethyl-14-oxa-benzo[α]naphthacen-7-one 234 (IVa).

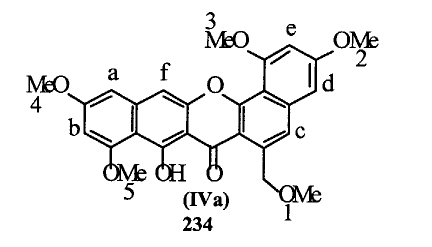

A solution of xanthone **228** (95 mg, 0.194 mmol) and lithium chloride (40 mg, 0.944 mmol) in DMF (2.8 mL) was stirred at 110°C for 13 hours. After being allowed to cool to room temperature, the mixture was diluted with water and then extracted with chloroform. The organic layer was washed several times with water and then brine, dried over anhydrous Na₂SO₄, filtered, and concentrated in vacuo to give xanthone 234 **(Iva)** as a red-brown solid (82 mg, 89 %).
mp 268-269°C;
IR (KBr) 3411, 2925, 1650, 1620, 1602, 1573 cm⁻¹;
¹H NMR (CDCl₃, 500 MHz) δ 7.74 (s, 1H, He), 7.10 (s, 1H, Hf), 6.80 (d, *J=* 2 Hz, 1H, Hd), 6.66 (d, *J=* 2.5 Hz, 1H, Ha), 6.61 (d, *J=* 2 Hz, 1H, He), 6.39 (d, *J=* 2 Hz, 1H, Hb), 5.26 (d, *J*= 1 Hz, 2H), 4.10 (s, 3H, C3), 4.03 (s, 3H, C5), 3.95 (s, 3H, C4), 3.95 (s, 3H, C2), 3.66 (s, 3H, C1);
¹³C NMR (CDCl₃, 75.4 MHz) δ 183.8 (C), 163.9 (C), 161.8 (C), 161.7 (C), 161.0 (C), 159.8 (C), 157.2 (C), 152.0 (C), 141.9 (C), 140.5 (C), 137.5 (C), 119.7 (CH), 112.4 (C), 109.7 (C), 108.2 (C), 104.4 (C), 100.8 (CH), 99.8 (CH), 99.6 (CH), 97.9 (CH), 96.9 (CH), 73.6 (CH₂), 59.1 (CH₃), 56.4 (CH₃), 56.3 (CH₃), 55.6 (CH₃), 55.5 (CH₃);
MS (DO, NH₃ + isobutane) m/z 477 (MH⁺, 16), 102 (100).

### ● Synthesis of xanthene Va (R₁ = R₂ = R₄ = R₅ OMe and R₃ = CH₂OMe)

### 1,3,9,11-Tetramethoxy-6-methoxymethyl-7H-14-oxa-benzo[α]naphthacen-8-ol (250).

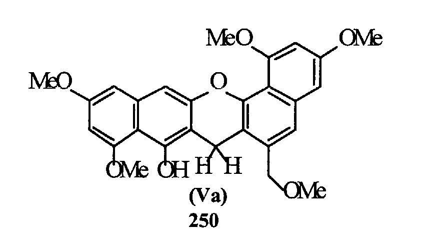

To a solution of xanthone **(IVa)** (37.5 mg, 0.079 mmol) in dry dichloromethane (7.9 mL) was added dropwise borane-methyl sulfide complex (75 µl, 0.791 mmol). The mixture was refluxed for 48 hours, and then allowed to cool to room temperature, diluted with an aqueous solution of HCl (0.5N), and extracted with dichloromethane. After the usual workup, xanthene **250 (Va)** (37.1 mg) was obtained, which was used without further purification.
¹H NMR (CDCl₃, 300 MHz) δ 7.39 (s, 1H), 6.94 (s, 1H), 6.69 (d, *J=* 2.4 Hz, 1H), 6.63 (d, *J=* 2.1 Hz, 1H), 6.53 (d, *J=* 2.4 Hz, 1H), 6.32 (d, *J=* 2 Hz, 1H), 4.63 (s, 2H), 4.07 (s, 2H), 4.02 (s, 3H), 4.01 (s, 3H), 3.88 (s, 3H), 3.87 (s, 3H), 3.48 (s, 3H);
MS (DCI, NH₃ + isobutane) m/z 461 (18), 197 (100).

### ● Synthesis of hypoxyxylenone derivative Ia (R₁ = R₂ = R₄ = R₅ = OMe and R₃ = CH₂OMe)

### Trimethyl-(1,3,6,9,11-tetramethoxy-6-methoxymethyl-7H-14-oxa-benzo[α] naphthacen-8-yloxy)-silane (258).

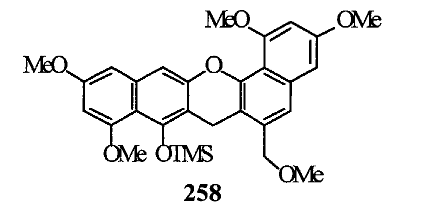

To a stirred solution of crude product 250 **(Va)** (37.1 mg) in anhydrous dichloromethane (0.85 mL) at 0°C were added dropwise 2,6-lutidine (64 µL, 0.552 mmol) and then trimethylsilyl trifluoromethanesulfonate (71 µL, 0.394 mmol). After 2.5 hours, the reaction mixture was quenched by addition of saturated aqueous NH₄Cl and then extracted with dichloromethane. The organic layer was washed with water, saturated aqueous CuSO₄, and brine, dried over Na₂SO₄, filtered, and concentrated under reduced pressure to afford xanthene **258** (42.3 mg) as a green substance, which was used without further purification.
¹H NMR (CDCl₃, 300 MHz) δ 7.36 (s, 1H), 7.10 (s, 1H), 6.68 (d, *J*= 2.3 Hz, 1H), 6.62 (d, *J=* 2.3 Hz, 1H), 6.54 (d, *J=* 2.3 Hz, 1H), 6.32 (d, *J=* 2.3 Hz, 1H), 4.59 (s, 2H), 4.09 (s, 2H), 4.03 (s, 3H), 3.88 (s, 9H), 3.45 (s, 3H), 0.28 (s, 9H).

### 1,3,9,11-Tetramethoxy-6-methoxymethyl-14-oxa-benzo[α]naphthacen-8-one 251 (Ia).

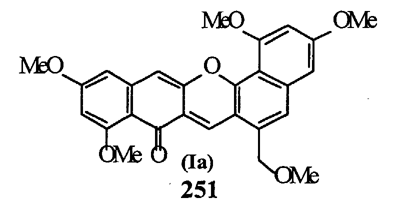

A solution of the above crude product **258** (42.3 mg) and palladium (II) acetate (17.5 mg, 0.078 mmol) in anhydrous acetonitrile (1.32 mL) was refluxed for 12 hours. After being allowed to cool to room temperature, the reaction mixture was concentrated in vacuo and the residue was immediately purified by flash column chromatography (silica gel treated with triethylamine 2.5 % w/v, dichloromethane and then dichloromethane-methanol 97:3) to give **251 (Ia)** as a blue substance (20.4 mg, 56 % for three steps).
IR (neat) 2926, 2855, 1659, 1613, 1590, 1536 cm⁻¹;
UV λmax (CH₃CN)/ nm 208 (log ε, 5.24), 234 (5.27), 323 (4.91), 610 (4.45);
¹H NMR (CDCl₃, 300 MHz) δ 8.43 (s,1H), 7.32 (s, 1H), 6.64 (d, *J=* 1.9 Hz, 1H), 6.50 (d, *J=* 2 Hz, 1H), 6.40 (d, *J=* 2.3 Hz, 1H), 6.26 (d, *J=* 2.2 Hz, 1H), 6.21 (s, 1H), 4.70 (s, 2H), 4.00 (s, 3H), 3.94 (s, 3H), 3.88 (s, 3H), 3.88 (s, 3H), 3.49 (s, 3H);
¹³C NMR (CDCl₃, 75.4 MHz) δ 178.6 (C), 164.3 (C), 163.9 (C), 161.1 (C), 159.3 (C), 153.5 (C), 150.7 (C), 143.9 (C), 139.3 (C), 133.8 (C), 133.2 (CH), 123.7 (C), 122.2 (CH), 113.4 (C), 112.2 (C), 109.9 (C), 101.9 (CH), 99.8 (CH), 99.5 (CH), 96.8 (CH), 95.1 (CH), 72.1 (CH₂), 58.7 (CH₃), 56.1 (CH₃), 55.9 (CH₃), 55.4 (CH₃), 55.4 (CH₃);
MS (DCI, NH₃ + isobutane) m/z 499 (7), 461 (MH⁺, 100);
HRMS calcd for C₂₇H₂₅O₇: 461.1600. Found : 461.1605 (MH⁺).

### Example 2

### - Synthesis of products of formula (TVb) wherein R₁ = R₂ = R'₃ = R₄ = R₅ = H ; R₁ = R₂ = OMe and R'₃ = R₄ = R₅ =H; R₁ = R₂ = R₄ = R₅ = OMe and R'₃ = Me ; R₁ = R₂ = R₄ = R₅ = OMe and R'₃ = CH₂OMe ; R₁ = R₂ = R₄ = R₅ = OH and R'₃ = Me. Synthesis of product of formula (V) wherein R₁ = R₂ = R₄ = R₅ = OMe and R"₃ = Me. Synthesis of product of formula (Ib) wherein R₁ = R₂ = R₄ = R₅ = OMe and R'₃ = Me.

### ● Synthesis of xanthones (IVb)

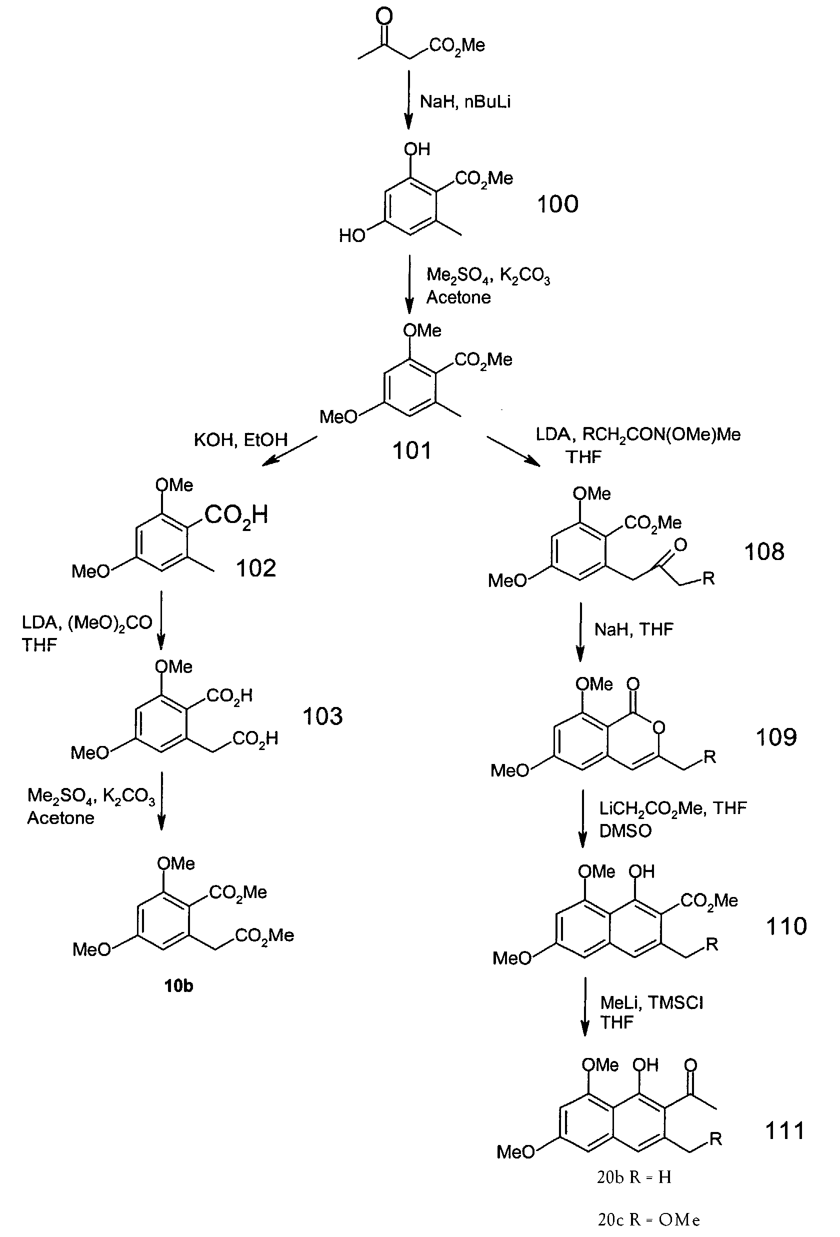

Product 10 b is one of the products of general formula (VI)
Product 20 b, c are products of general formula (VII) 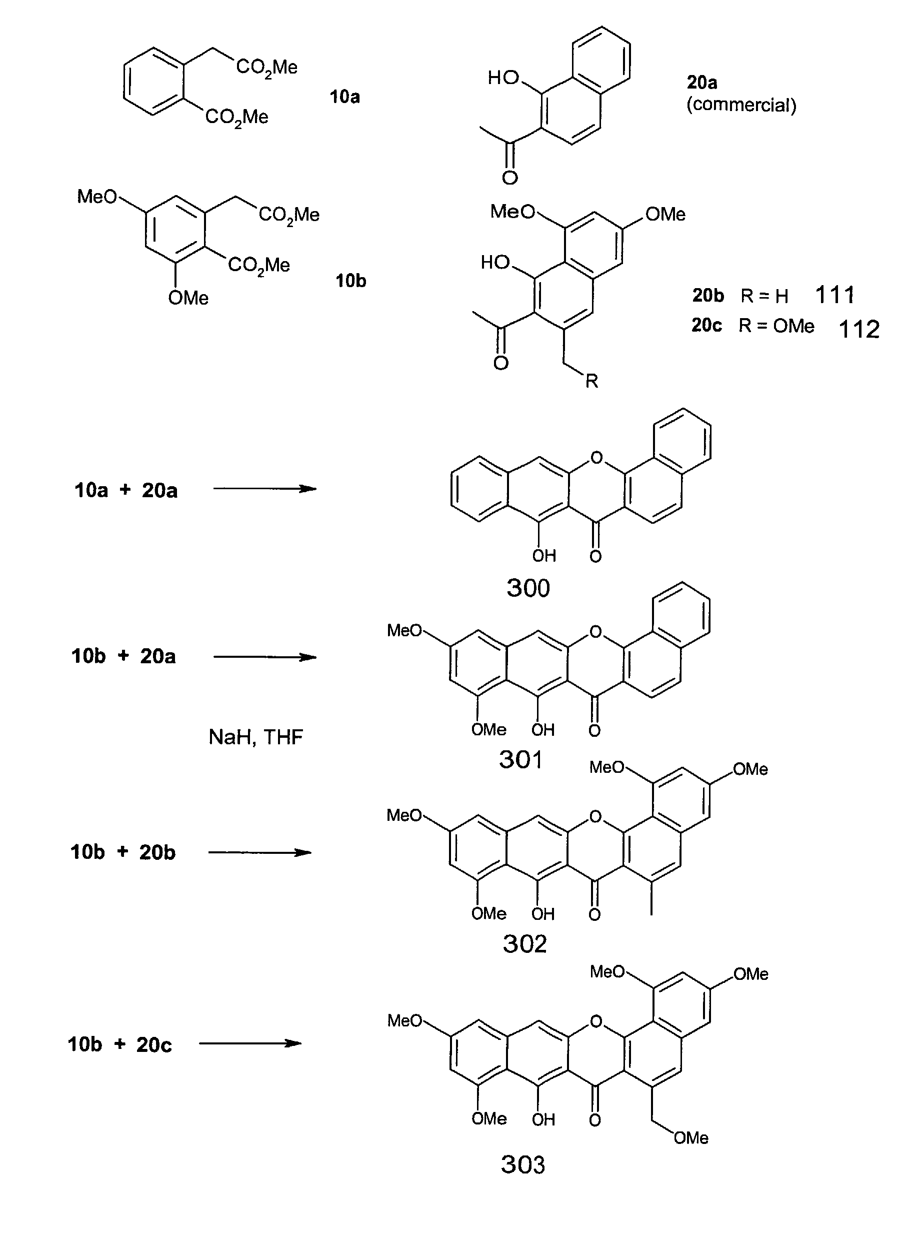 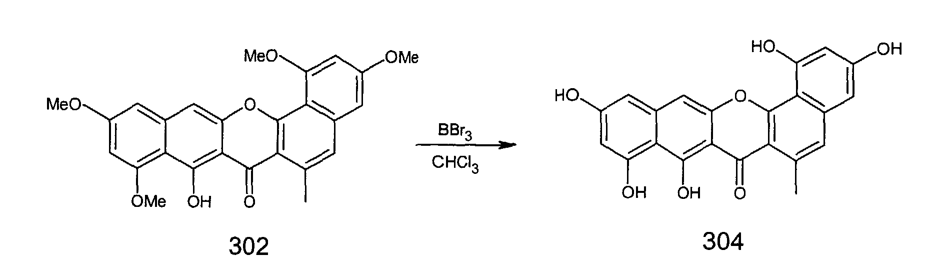 Product 10a is one of the products of general formula (VI)
Product 20a is one of the products of general formula (VII)
Product 300-304 are products of general formula (IVb)

### ● Synthesis of xanthene derivative of formula (V). Synthesis of hypoxyxylerone derivative of formula (Ib)

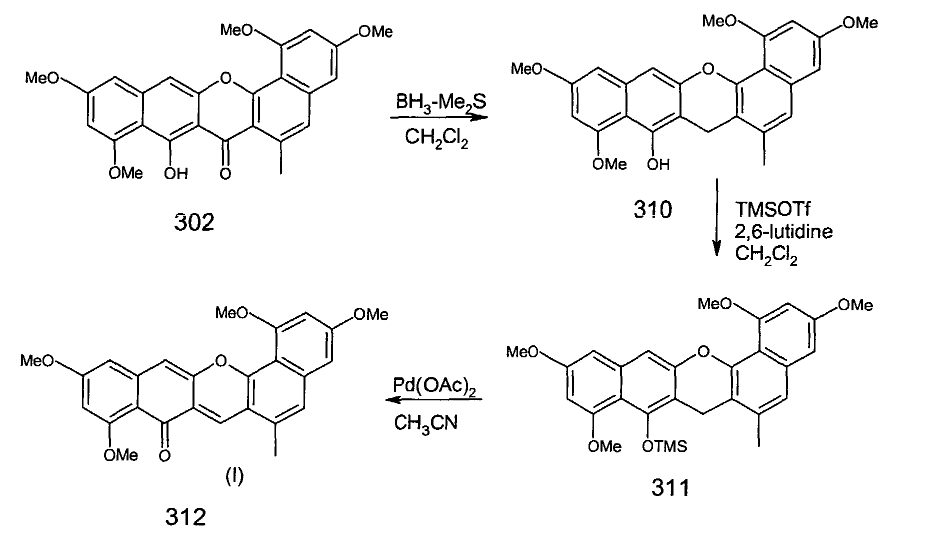

Product 310 is one of the products of general formula (V)
Product 312 is one of the products of general formula (Ib)

### ● Synthesis of xanthones of formula (IVb)

### 2,4-Dimethoxy-6-methyl-benzoic acid methyl ester (101)

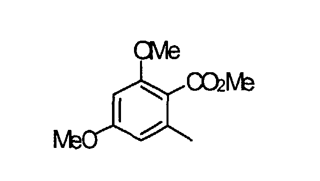

A mixture of 2,4-dihydroxy-6-methyl benzoic acid methyl ester **100** (4 g, 22 mmol) and dry potassium carbonate (7.6 g, 55 mmol) in anhydrous acetone (70 mL) and dimethyl sulfate (5.2 mL, 55 mmol) was heated to reflux under argon for 16 hours. The mixture was cooled, filtered to remove potassium carbonate, which was washed with acetone. The filtrate was evaporated and the residue was dissolved in diethyl ether, washed several times with aqueous NaOH (4M). After the usual workup, the product was chromatographed (flash silica gel, pentane-EtOAc 8:2) to give ester **101** (4.5 g, 97 %) as a white solid.
mp 44-45 °C ;
IR (neat) 3000, 2947, 2841, 1729, 1606, 1589 cm⁻¹;
¹H NMR (CDCl₃, 300 MHz) δ 6.29 (s, 2H), 3.86 (s, 3H), 3.78 (s, 3H), 3.77 (s, 3H), 2.26 (s, 3H);
¹³C NMR (CDCl₃, 75.4 MHz) δ 168.8 (C), 161.5 (C), 158.3 (C), 138.4 (C), 116.5 (C), 106.8 (CH), 96.3 (CH), 56.0 (CH₃), 55.5 (CH₃), 52.1 (CH₃), 20.1 (CH₃).
Spectral properties identical to samples already described in literature.

### 2,4-Dimethoxy-6-methyl-benzoic acid (102).

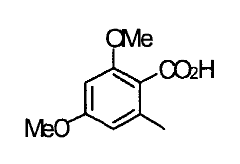

Ester **101** (2.1 g, 10 mmol) was refluxed in a 10 % solution of KOH in ethanol-water 95:5 (85 mL) for 16 hours. The mixture was cooled and concentrated in vacuo. The resultant residue was acidified to pH 1 with aqueous HCl (6N) and extracted with ethyl acetate. After the usual workup, the carboxylic acid **102** (1.94 g, 99 %) was obtained as a yellow solid.
mp 140-142°C;
IR (neat) 2954, 2848, 2647, 1684, 1603, 1583 cm⁻¹;
¹H NMR (CDCl₃, 300 MHz) δ 10.69 (s, 1H), 6.42 (d, *J=* 2.2 Hz, 1H), 6.37 (d, *J=* 2.2 Hz, 1H) 3.93 (s, 3H), 3.82 (s, 3H), 2.56 (s, 3H);
¹³C NMR (CDCl₃, 75.4MHz) δ 171.2 (C), 161.9 (C), 159.1 (C), 141.2 (C), 113.9 (C), 108.0 (CH), 96.2 (CH), 56.1 (CH₃), 55.2 (CH₃), 21.3 (CH₃).
Spectral properties identical to samples already described in literature.

### 2-Carboxymethyl-4,6-dimethoxy-benzoic acid (103).

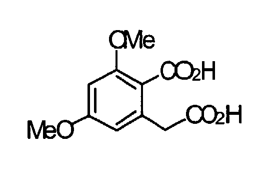

To a solution of diisopropylamine (4.7 mL, 33.6 mmol) in anhydrous THF (11 mL) under argon at 0°C was added n-butyllithium (2.5 molar in hexane, 12.8 mL, 32 mmol). The solution was stirred for 10 minutes, cooled to -78°C and a mixture of carboxylic acid **102** (1.57 g, 8 mmol) and dimethylcarbonate (1.7 mL, 20 mmol) in THF (11 mL) was added dropwise over 10 minutes. When the addition was completed, the cooling bath was removed, the reaction was allowed to warm to room temperature and stirred for 4 hours. Water (11 mL) was then added and stirring was continued overnight. The solution was concentrated in vacuo, the resultant residue was acidified to pH 1 with aqueous HCl (6N) and then extracted with hot ethyl acetate. After the usual workup, the crude product was triturated with diethyl ether and filtered to give homophthalic acid **103** (1.44 g, 75 %) as a white solid.
mp 182-183°C ;
IR (KBr) 3026, 2951, 2849, 2636, 1713, 1657, 1605, 1576 cm⁻¹;
¹H NMR (CD₃COCD₃, 300 MHz) δ 6.59 (d, *J*= 2.3 Hz, 1H), 6.56 (d, *J*= 2.3 Hz, 1H) 3.89 (s, 3H), 3.85 (s, 3H), 3.78 (s, 2H);
¹³C NMR (CD₃COCD₃, 75.4 MHz) δ 172.0 (C), 168.3 (C), 162.8 (C), 160.0 (C), 137.8 (C), 116.5 (C), 109.6 (CH), 98.1 (CH), 56.6 (CH₃), 55.9 (CH₃), 40.0 (CH₂);
Spectral properties identical to samples already described in literature.

### 2,4-Dimethoxy-6-methoxycarbonylmethyl-benzoic acid methyl ester (10b) (VI).

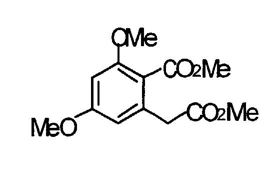

A mixture of homophthalic acid **103** (1.2 g, 5 mmol) and dry potassium carbonate (1.73 g, 12.5 mmol) in anhydrous acetone (25 mL) and dimethyl sulfate (1.2 mL, 12.5 mmol) was heated to reflux under argon for 16 hours. The mixture was cooled, filtered to remove potassium carbonate, which was washed with acetone. The filtrate was evaporated and the residue was dissolved in diethyl ether, washed twice with aqueous NaOH (4M). After the usual workup, the product was chromatographed (flash silica gel, pentane-EtOAc 7:3) to give diester **10b** (1.15 g, 86 %) as a white solid.
mp 54-55°C ;
IR (neat) 3003, 2952, 2842, 1732, 1713, 1608, 1586 cm⁻¹;
¹H NMR (CDCl₃, 300 MHz) δ 6.38 (d, *J=* 2.2 Hz, 1H), 6.37 (d, *J=* 2.2 Hz, 1H), 3.83 (s, 3H), 3.79 (s, 3H), 3.78 (s, 3H), 3.65 (s, 3H), 3.63 (s, 2H);
¹³C NMR (CDCl₃, 75.4 MHz) δ 171.1 (C), 167.7 (C), 161.8 (C), 159.0 (C), 135.1 (C), 115.9 (C), 107.6 (CH), 97.8 (CH), 56.0 (CH₃), 55.4 (CH₃), 52.0 (CH₃), 51.9 (CH₃), 39.6 (CH₂).
Spectral properties identical to samples already described in literature.

### 2-methoxycarbonylmethyl-benzoic acid methyl ester 10a (VI).

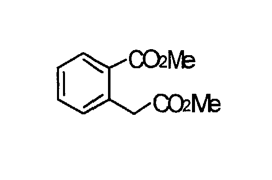

Reaction of homophthalic acid (2.5 g, 13.9 mmol) as outlined above yielded diester **10a** (2.72 g, 94 %) as a colourless oil.
IR (neat) 3067, 3005, 2954, 2844, 1742, 1723, 1607, 1580 cm⁻¹;
¹H NMR (CDCl₃, 300 MHz) δ 7.99 (dd, *J=* 7.7 Hz, *J=* 1.5 Hz, 1H), 7.44 (td, *J=* 7.7 Hz, *J=* 1.5 Hz, 1H), 7.32 (td, *J=* 7.7 Hz, *J=* 1.5 Hz, 1H), 7.23 (dd, *J=* 7.7 Hz, *J*= 0.9 Hz, 1H), 3.99 (s, 2H), 3.83 (s, 3H), 3.66 (s, 3H);
¹³C NMR (CDCl₃, 75.4 MHz) δ 171.9 (C), 167.4 (C), 136.0 (C), 132.3 (CH), 132.2 (CH), 131.0 (CH), 129.6 (C), 127.4 (CH), 51.9 (CH₃), 51.8 (CH₃), 40.4 (CH₂).
Spectral properties identical to samples already described in literature.

### N-Methoxy-N-methyl-acetamide (105).

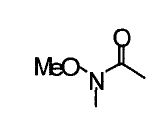

Pyridine (10 mL, 124 mmol) was slowly added to a well stirred slurry of O,N-dimethylhydroxylamine hydrochloride (6 g, 62 mmol) and acetyl chloride (4.15 mL, 59 mmol) in CH₂Cl₂ (85 mL) under argon at O°C. The mixture was warmed to room temperature, stirred for 3 hours and then partitioned between brine (55 mL) and diethyl ether (85 mL). Extraction with additional diethyl ether, drying of the combined organic layers over anhydrous Na₂SO₄, concentration, followed by distillation under reduce pressure gave Weinreb amide **105** (4.54 g, 72 %) as a colourless liquid.
bp 42-44°C / 20 mmHg ;
IR (neat) 3497, 2971, 2941, 2824, 1663 cm⁻¹;
¹H NMR (CDCl₃, 300 MHz) δ 3.62 (s, 3H), 3.11 (s, 3H), 2.05 (s, 3H);
¹³C NMR (CDCl₃, 75.4 MHz) δ 171.8 (C), 61.0 (CH₃), 31.9 (CH₃), 19.6 (CH₃).
Spectral properties identical to samples already described in literature.

### 2,N-Dimethoxy-N-methyl-acetamide (106).

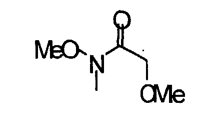

Reaction of O,N-dimethylhydroxylamine hydrochloride (6 g, 62 mmol) with methoxy-acetyl chloride (5.4 mL, 59 mmol) as outlined above yielded Weinreb amide **106** (4.11 g, 52 %) as a colourless liquid.
bp 62-64°C / 2 mmHg ;
IR (neat) 3498, 2970, 2939, 2825, 1679 cm⁻¹;
¹H NMR (CDCl₃, 300 MHz) δ 4.19 (s, 2H), 3.66 (s, 3H), 3.43 (s, 3H), 3.16 (s, 3H);
¹³C NMR (CDCl₃, 75.4 MHz) δ 170.6 (C), 69.5 (CH₂), 61.1 (CH₃), 59.0 (CH₃), 32.0 (CH₃).
MS (DCI, NH₃+isobutane) m/z 134 (MH⁺, 100), 103 (10), 74 (11).

### 2,4-Dimethoxy-6-(2-oxo-propyl)-benzoic acid methyl ester (107).

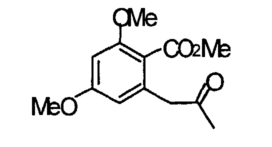

A solution of diisopropylamine (1.7 mL, 12 mmol) and n-butyllithium (2.4 molar in hexane, 4.8 mL, 11.5 mmol) in anhydrous THF (30 mL) was stirred under argon at room temperature for 10 minutes, and then cooled to -78°C. Ester **101** (2.1 g, 10 mmol) dissolved in dry THF (30 mL) is added, and the resulting red/orange solution stirred for 20 minutes before addition of Weinreb amide **105** (1.2 g, 11.5 mmol). The solution was stirred at -78°C for 90 minutes and then aqueous HCl (2N, 30 mL) was added. The mixture was allowed to warm to room temperature over 2 hours, diluted with further aqueous HCl (2N, 30 mL) and extracted with ethyl acetate. After the usual workup, the crude oil was chromatographed (flash silica gel, pentane-EtOAc 6:4) to give keto-ester **107** (1,87 g, 74 %) as a yellow solid.
mp 61-62°C ;
IR (neat) 3007, 2952, 2842, 1733, 1714, 1606, 1587 cm⁻¹;
¹H NMR (CDCl₃, 300 MHz) δ 6.34 (d, *J=* 2.2 Hz, 1H), 6.25 (d, *J=* 2.2 Hz, 1H), 3.77 (s, 3H), 3.74 (s, 3H), 3.73 (s, 3H), 3.61 (s, 2H), 2.07 (s, 3H);
¹³C NMR (CDCl₃, 75.4 MHz) δ 205.2 (C), 167.9 (C), 161.8 (C), 159.1 (C), 135.8 (C), 115.9 (C), 107.5 (CH), 97.6 (CH), 55.9 (CH₃), 55.3 (CH₃), 51.9 (CH₃), 49.0 (CH₂), 29.2 (CH₃);
Spectral properties identical to samples already described in literature.

### 2,4-Dimethoxy-6-(3-methoxy-2-oxo-propyl)-benzoic acid methyl ester (108).

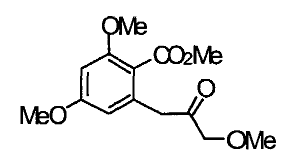

Reaction of ester 101 (2.1 g, 10 mmol) with Weinreb amide **106** (1.53 g, 11.5 mmol) as outlined above yielded keto-ester **108** (1.92 g, 68 %) as an oil.

IR (neat) 3005, 2953, 2844, 1729, 1604, 1587 cm⁻¹;
¹H NMR (CDCl₃, 300 MHz) δ 6.33 (d, *J=* 2.2 Hz, 1H), 6.26 (d, *J=* 2.2 Hz, 1H), 4.00 (s, 2H), 3.76 (s, 3H), 3.73 (s, 3H), 3.72 (s, 3H), 3.66 (s, 2H), 3.32 (s, 3H);
¹³C NMR (CDCl₃, 75.4 MHz) δ 204.6 (C), 167.9 (C), 161.8 (C), 159.2 (C), 135.1 (C), 115.7 (C), 107.8 (CH), 97.7 (CH), 76.9 (CH₂), 59.2 (CH₃), 55.9 (CH₃), 55.3 (CH₃), 52.0 (CH₃), 44.6 (CH₂);
MS (DCI, NH₃+isobutane) m/z 283 (MH⁺, 100), 251 (62), 225 (75), 209 (78), 193 (78);
Anal. calcd for C₁₄H₁₈O₆: C, 59.57; H, 6.43. Found: C, 59.58; H, 6.58.

### 6,8-Dimethoxy-3-methyl-2H-naphthalen-1-one (109).

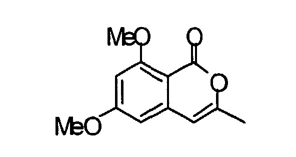

To a mixture of sodium hydride (240 mg, 6 mmol as a 60 % dispersion in mineral oil) washed several times with pentane, in anhydrous THF (80 mL) and t-butyl alcohol (one drop) under argon at 0°C, was added dropwise a solution of keto-ester **107** (1.26 g, 5 mmol) in THF (40 mL). The resulting mixture was stirred at 0°C for 15 minutes and at room temperature until no starting material remained in thin-layer chromatography. Excess of base was carefully hydrolysed at 0°C by addition of saturated aqueous NH₄Cl and the mixture was extracted with ethyl acetate. After the usual workup, isocoumarin **109** (1.09 g, 99 %) was obtained as a white solid and used without further purification.
mp 150-151°C (ethanol);
IR (neat) 3095, 2915, 2848, 1718, 1658, 1598, 1568 cm⁻¹;
¹H NMR (CDCl₃, 300 MHz) δ 6.38 (d, *J=* 2.2 Hz, 1H), 6.27 (d, *J=* 2.2 Hz, 1H), 6.06 (s, 1H), 3.93 (s, 3H), 3.86 (s, 3H), 2.19 (d, *J=* 0.8 Hz, 3H);
¹³C NMR (CDCl₃, 75.4 MHz) δ 165.1 (C), 162.9 (C), 159.2 (C), 155.1 (C), 142.1 (C), 103.4 (CH), 102.3 (C), 99.3 (CH), 97.8 (CH), 55.9 (CH₃), 55.3 (CH₂), 19.1 (CH₃);
Spectral properties identical to samples already described in literature.

### 6,8-Dimethoxy-3-methoxymethyl-2H-naphthalen-1-one (110).

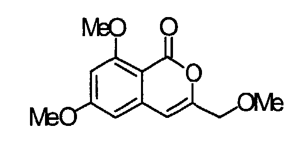

Reaction of keto-ester **108** (1.41 g, 5 mmol) as outlined above yielded isocoumarine **110** (1.24 g, 99 %) as a white solid.
mp 110-111°C (ethanol);
IR (neat) 3095, 2941, 2847, 1729, 1672, 1598, 1574 cm⁻¹;
¹H NMR (CDCl₃, 300 MHz) δ 6.43 (d, *J=* 2.2 Hz, 1H), 6.35 (d, *J=* 2.2 Hz, 1H), 6.34 (s, 1H), 4.19 (d, *J=* 0.9 Hz, 2H), 3.93 (s, 3H), 3.87 (s, 3H), 3.45 (s, 3H);
¹³C NMR (CDCl₃, 75.4 MHz) δ 165.5 (C), 163.4 (C), 158.8 (C), 154.6 (C), 141.6 (C), 103.5 (CH), 103.3 (C), 100.4 (CH), 98.7 (CH), 70.3 (CH₂), 59.1 (CH₃), 56.3 (CH₃), 55.7 (CH₃);
MS (DCI, NH₃+isobutane) m/z 251 (MH⁺, 100), 205 (12);
Anal. calcd for C₁₃H₁₄O₅: C, 62.39; H, 5.64. Found: C, 62.23; H, 5.71.

### 1-Hydroxy-6,8-dimethoxy-3-methyl-naphthalene-2-carboxylic acid methyl ester (111)

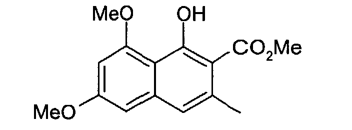

A solution of diisopropylamine (10.5 mL, 75 mmol) and n-butyllithium (1.8 molar in hexane, 40 mL, 72 mmol) in anhydrous THF (175 mL) was stirred under argon for 20 minutes at 0°C, 5 minutes at room temperature and then cooled to -78°C. Methyl acetate (6 mL, 75 mmol) was added and then 30 minutes later, the solution of methyl lithioacetate was transferred rapidly by cannula to a solution of isocoumarine (660 mg, 3 mmol) dissolved in DMSO (90 mL) and THF (90 mL) at 0°C under argon. The solution which immediately turned yellow, was stirred for 15 minutes at 0°C, and after 15 minutes at room temperature, the mixture was treated with AcOH (150 mL) and stirred for 48 hours. The solution was partially concentrated in vacuo, diluted with diethyl ether and the organic layer was successively washed with saturated aqueous NaHCO₃ and water. After the usual workup, the crude yellow liquid was chromatographed (flash silica gel, pentane-EtOAc 8:2) to give methyl naphthoate **111** (696 mg, 84 %) as a yellow solid.
mp 84-85°C (diethyl ether);
IR (neat) 3381, 2954, 2851, 1724, 1633, 1620, 1588 cm⁻¹;
¹H NMR (CDCl₃, 300 MHz) δ 10.68 (s, 1H), 6.94 (s, 1H), 6.56 (d, *J=* 2.2 Hz, 1H), 6.40 (d, *J=* 2.2 Hz, 1H), 3.97 (s, 3H), 3.93 (s, 3H), 3.86 (s, 3H), 2.42 (s, 3H);
¹³C NMR (CDCl₃, 75.4 MHz) δ 170.0 (C), 159.2 (C), 157.9 (C), 155.7 (C), 138.1 (C), 135.1 (C), 118,8 (CH), 111.9 (C), 108.7 (C), 98.4 (CH), 97.3 (CH), 55.8 (CH₃), 54.9 (CH₃), 51.6 (CH₃), 21.0 (CH₃);
Spectral properties identical to samples already described in literature.

### 1-Hydroxy-6,8-dimethoxy-3-methoxymethyl-naphthalene-2-carboxylic acid methyl ester (112)

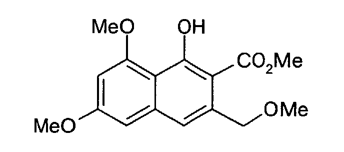

Reaction of isocoumarin **110** (750 mg, 3 mmol) as outlined above yielded methyl naphthoate **112** (687 mg, 75 %) as a yellow solid.
mp 93-94°C (diethyl ether);
IR (neat) 3375, 2949, 2852, 1722, 1633, 1621, 1592 cm⁻¹;
¹H NMR (CDCl₃, 300 MHz) δ 10.66 (s, 1H), 7.13 (s, 1H), 6.59 (d, *J=* 2.2 Hz, 1H), 6.38 (d, *J=* 2.2 Hz, 1H), 4.57 (s, 2H), 3.91 (s, 3H), 3.89 (s, 3H), 3.80 (s, 3H), 3.35 (s, 3H);
¹³C NMR (CDCl₃, 75.4 MHz) δ 169.6 (C), 159.7 (C), 158.3 (C), 156.5 (C), 138.3 (C), 136.0 (C), 116,9 (CH), 110.0 (C), 109.9 (C), 99.6 (CH), 98.4 (CH), 73.2 (CH₂), 58.3 (CH₃), 56.3 (CH₃), 55.3 (CH₃), 52.0 (CH₃);
MS (DCI, NH₃+isobutane) m/z 307 (MH⁺, 100), 275 (59);
Anal. calcd for C₁₆H₁₈O₆: C, 62.74; H, 5.92. Found: C, 62.53; H, 5.96.

### 1-(1-Hydroxy-6,8-dimethoxy-3-methyl-naphthalen-2-yl) ethanone 20b (VII).

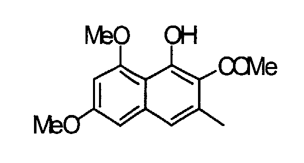

A stirred solution of methyl naphthoate **111** (400 mg, 1.45 mmol) in anhydrous THF (16 mL) under argon at 0°C was treated rapidly with methyllithium (4.15 mL of a 1.4 M solution in diethyl ether, 5.8 mmol). After 2 hours at 0°C, freshly distilled Me₃SiCl (3.7 mL, 29 mmol) was rapidly added while stirring continued. The reaction mixture was then allowed to come to room temperature over 30 minutes, at which point 16 mL of aqueous HCl (IN) was added and the resulting two-phase system was stirred for 30 minutes. After extraction with diethyl ether and the usual workup, the crude yellow liquid was chromatographed (flash silica gel, pentane-EtOAc 8:2) to give acetonaphthone **20b** (235 mg, 62 %) as a yellow solid.
mp 94-95°C (diethyl ether);
IR (neat) 3363, 2923, 2845, 1687, 1678, 1632, 1588 cm⁻¹;
¹H NMR (CDCl₃, 300 MHz) δ 9.74 (s, 1H), 6.91 (s, 1H), 6.53 (d, *J*= 2.2 Hz, 1H), 6.35 (d, *J=* 2.2 Hz, 1H), 3.94 (s, 3H), 3.83 (s, 3H), 2.58 (s, 3H), 2.32 (s, 3H);
¹³C NMR (CDCl₃, 75.4 MHz) δ 205.1 (C), 159.0 (C), 157.7 (C), 153.6 (C), 137.8 (C), 135.1 (C), 122,5 (C), 119.2 (CH), 108.8 (C), 99.0 (CH), 97.6 (CH), 56.2 (CH₃), 55.4 (CH₃), 32.4 (CH₃), 20.4 (CH₃);
Spectral properties identical to samples already described in literature.

### 1-(1-Hydroxy-6,8-dimethoxy-3-methoxymethyl-naphthalen-2-yl) ethanone 20c (VII).

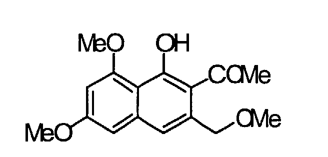

Reaction of methyl naphthoate **112** (400 mg, 1.3 mmol) as outlined above yielded acetonaphthone **20c** (223 mg, 59 %) as a yellow solid.
mp 96-97°C (diethyl ether);
IR (neat) 3370, 2927, 2858, 1695, 1633, 1595 cm⁻¹;
¹H NMR (CDCl₃, 300 MHz) δ 9.65 (s, 1H), 7.14 (s, 1H), 6.63 (d, *J*= 2.2 Hz, 1H), 6.42 (d, *J=* 2.2 Hz, 1H), 4.51 (s, 2H), 3.98 (s, 3H), 3.84 (s, 3H), 3.35 (s, 3H), 2.60 (s, 3H);
¹³C NMR (CDCl₃, 75.4 MHz) δ 205.0 (C), 159.1 (C), 157.7 (C), 153.5 (C), 137.6 (C), 135.9 (C), 121,0 (C), 117.4 (CH), 109.8 (C), 99.8 (CH), 98.4 (CH), 72.8 (CH₂), 58.5 (CH₃), 56.4 (CH₃), 55.5 (CH₃), 32.3 (CH₃);
MS (DCI, NH₃+isobutane) m/z 291 (MH⁺, 100), 110 (40);
Anal. calcd for C₁₆H₁₈O₅: C, 66.20; H, 6.25. Found: C, 65.98; H, 6.27.

### ●● Synthesis of xanthone (IVb) (R₁ = R₂ = R'₃ = R₄ = R₅ = H)

### 8-Hydroxy-14-oxa-benzo[α]naphthacen-7-one 300 (IVb)

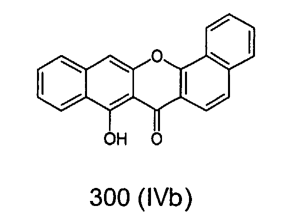

To a stirred suspension of sodium hydride (161 mg, 4 mmol as a 60 % dispersion in mineral oil) washed several times with pentane in anhydrous THF (2.5 mL) under argon at 0°C, was added dropwise a solution of 1-hydroxy-2-acetonaphthone (150 mg, 0.8 mmol) in THF (5 mL). The resulting mixture was stirred at 25°C for 1 hour before addition of a solution of homophthalic acid dimethyl ester (285 mg, 1.36 mmol) in THF (3.5 mL). The yellow/orange mixture was stirred at 25°C for 2 hours and then heated to reflux for 20 hours. The red solution was cooled, aqueous HCl (6N) was added and the mixture was extracted with chloroform. After the usual workup, the product was chromatographed (flash silica gel, pentane-EtOAc 9:1) to give dibenzoxanthone 300 (IVb)(174 mg, 69 %) as an orange-coloured solid.
mp 228-229°C (chloroform);
IR (neat) 3403, 3060, 2960, 2919, 2850, 1652, 1625, 1603, 1571 cm⁻¹;
¹H NMR (CDCl₃, 300 MHz) δ 8.68 (m, 1H), 8.45 (dd, *J=* 8.5 Hz, *J=* 0.6 Hz, 1H), 8.19 (d, *J=* 8.5 Hz, 1H), 7.92 (m, 1H), 7.82 (d, *J=* 8.5 Hz, 1H), 7.76-7.60 (m, 4H), 7.50-7.43 (m, 2H);
¹³C NMR (CDCl₃, 75.4 MHz) δ 183.1 (C), 161.8 (C), 154.8 (C), 151.2 (C), 137.4 (C), 137.2 (C), 130.6 (CH), 130.1 (CH), 128.2 (CH), 127.1 (CH), 127.0 (CH), 124.3 (CH), 124.1 (CH), 123.9 (C), 123.7 (CH), 123.3 (CH), 120.7 (C), 120.6 (CH), 115.4 (C), 105.1 (C), 102.3 (CH);
MS (DCI, NH₃+isobutane) m/z 313 (MH⁺, 100);
HRMS calcd for C₂₁H₁₂O₃: 312.0786. Found: 312.0794 (M⁺).

### ●● Synthesis of xanthone (IVb) (R₁ = R₂ = OMe and R'₃ = R₄ = R₅ = H)

### 8-Hydroxy-9,11-dimethoxy-14-oxa-benzo[α]naphthacen-7-one 301 (IVb).

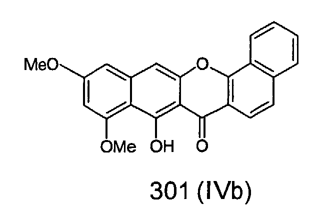

Reaction of 1-hydroxy-2-acetonaphthone (150 mg, 0.8 mmol) with diester **10b** (365 mg, 1.36 mmol) as outlined above yielded, after purification by silica gel chromatography (pentane-EtOAc 8:2), dibenzoxanthone 301 (IVb) (138 mg, 46 %) as an orange-coloured solid.
mp 253-254°C (chloroform);
IR (neat) 3376, 3008, 2971, 2919, 2852, 1650, 1614, 1598, 1573 cm⁻¹;
¹H NMR (CDCl₃, 300 MHz) δ 8.62 (m, 1H), 8.18 (d, *J=* 8.7 Hz, 1H), 7.90 (m, 1H), 7.73-7.62 (m, 3H), 7.19 (s, 1H), 6.65 (d, *J=* 2.2 Hz, 1H), 6.38 (d, *J*= 2.2 Hz, 1H), 4.01 (s, 3H), 3.94 (s, 3H);
¹³C NMR (CDCl₃, 75.4 MHz) δ 182.4 (C), 164.2 (C), 162.1 (C), 161.0 (C), 154.1 (C), 152.4 (C), 142.0 (C), 137.0 (C), 129.9 (CH), 128.2 (CH), 127.0 (CH), 123.7 (C), 123.6 (CH), 123.1 (CH), 120.7 (CH), 115.6 (C), 108.2 (C), 104.1 (C), 101.3 (CH), 98.1 (CH), 97.2 (CH), 56.3 (CH₃), 55.6 (CH₃);
MS (DCI, NH₃+isobutane) m/z 373 (MH⁺, 100);
HRMS calcd for C₂₃H₁₆O₅: 372.0998. Found: 372.0995 (M⁺).

### ●● Synthesis of xanthone (IVb) (R₁ = R₂ = R₄ = R₅ = OMe and R'₃ = Me)

### (1,3-dihydroxy-6,8-dimethoxy-naphthalen-2-yl)-(1-hydroxy-6,8-dimethoxy-3-methyl-naphthalen-2-yl)-methanone and 8-Hydroxy-1,3,9,11-tetramethoxy-6-methyl-14-oxa-benzo[α]naphthacen-7-one 302 (IVb)

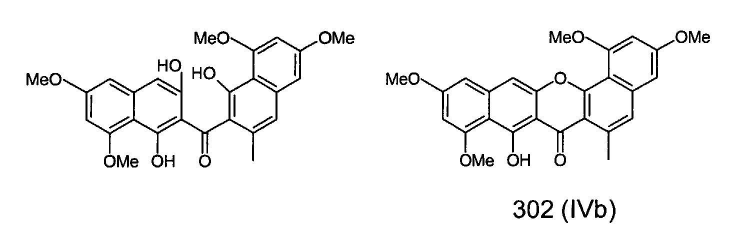

Reaction of acetonaphthone **20b** (170 mg, 0.65 mmol) with diester **10b** (300 mg, 1.11 mmol) as outlined above yielded, after purification by silica gel chromatography (pentane-EtOAc 7:3 and then 5:5), a mixture of dinaphthonaphthone and dibenzoxanthone **302 (TVb),** which was heated in water-toluene 5:1 (12 mL) at 200°C for 16 hours (pressure tube). After cooling, extraction with chloroform followed by the usual workup, the resulting solid product was triturated with diethyl ether and filtered to give dibenzoxanthone **302 (IVb)** (122 mg, 42 %) as an orange-coloured solid.
mp 263-264°C (chloroform);
IR (neat) 3412, 3006, 2969, 2924, 2852, 1648, 1619, 1599, 1563 cm⁻¹;
¹H NMR (CDCl₃, 300 MHz) δ 7.14 (s, 1H), 6.98 (s, 1H), 6.61 (d, *J=* 2.3 Hz, 1H), 6.60 (d, *J=* 2.3 Hz, 1H), 6.52 (d, *J=* 2.3 Hz, 1H), 6.34 (d, *J=* 2.3 Hz, 1H), 4.05 (s, 3H), 3.99 (s, 3H), 3.91 (s, 3H), 3.90 (s, 3H), 2.91 (s, 3H);
¹³C NMR (CDCl₃, 75.4 MHz) δ 184.4 (C), 164.4 (C), 161.9 (C), 161.8 (C), 161.4 (C), 160.0 (C), 157.4 (C), 152.3 (C), 142.0 (C), 140.5 (C), 137.3 (C), 124.5 (CH), 114.1 (C), 110.1 (C), 108.6 (C), 104.7 (C), 100.6 (CH), 99.6 (CH), 99.2 (CH), 98.3 (CH), 97.2 (CH), 56.5 (CH₃), 56.3 (CH₃), 55.6 (CH₃), 55.5 (CH₃), 23.9 (CH₃);
MS (DCI, NH₃+isobutane) m/z 447 (MH⁺, 100);
HRMS calcd for C₂₆H₂₂O₇: 446.1366. Found: 446.1346 (M⁺).

### ●● Synthesis of xanthone (IVb) (R₁ = R₂ = R₄ = R₅ = OMe and R'₃ = CH₂OMe

### (1,3-Dihydroxy-6,8-dimethoxy-naphthalen-2-yl)-(1-hydroxy-6,8-dimethoxy-3-methoxymethyl-naphthalen-2-yl)-methanone and 8-Hydroxy-1,3,9,11-tetramethoxy-6-methoxymethyl-14-oxa-benzo[α]naphthacen-7-one 303 (IVb)

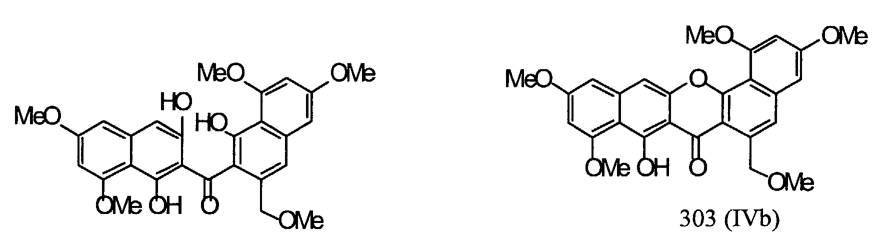

Reaction of acetonaphthone **20c** (130 mg, 0.45 mmol) with diester **10b** (204 mg, 0.76 mmol) as outlined above yielded, after purification by silica gel chromatography (pentane-EtOAc 7:3 and then 5:5), a mixture of dinaphthonaphthone and dibenzoxanthone, which was heated in water-toluene 5:1 (12 mL) at 200°C for 16 hours (pressure tube). After cooling, extraction with chloroform followed by the usual workup, the resulting solid product was triturated with diethyl ether and filtered to give dibenzoxanthone **303 (IVb)** (79 mg, 37 %) as an orange-coloured solid, the same as obtained by the first process. This material was transformed to the corresponding hypoxyxylerone derivative **(Ib)** (R₁ = R₂ = R₄ = R₅ = OMe and R'₃ = CH₂OMe) via the corresponding xanthene **(V)** (R₁ = R₂ = R₄ = R₅ = OMe and R'₃ = CH₂OMe).

### ●● Synthesis of xanthone (IVb) (R₁ = R₂ = R₄ = R₅ = OH and R'₃ = Me)

### 1, 3, 8, 9, 11-Pentahydroxy-6-methyl-14-oxa-benzo [α] naphthacen-7-one 304 (IVb)

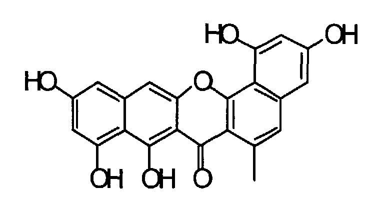

To a stirred suspension of dibenzoxanthone 302 (10 mg, 0.022 mmol) in anhydrous chloroform (0.7 mL) under argon at -5 °C, was added dropwise boron tribromide (335 µL of a 2 M freshly prepared solution in chloroform, 0.67 mmol). The mixture, which immediately turned purple, was allowed to warm slowly to room temperature, stirred in the dark for 24 hours and then treated under argon at 0°C with several drops of methanol. After evaporation in vacuo, additional methanol was added and then evaporated, until the release of gaseous hydrobromic acid terminated. The crude product was immediately purified by flash column chromatography (silica gel, dichloromethane-methanol 95:5) to give **304 (IVb)** (4.3 mg, 49 %) as a brown solid.
¹H NMR (CD₃COCD₃, 300 MHz) δ 7.11 (s, 1H), 6.69 (d, *J*= 2.2 Hz, 1H), 6.67 (s, 1H), 6.61 (d, *J*= 2.2 Hz, 1H), 6.45 (d, *J*= 2.2 Hz, 1H), 6.09 (d, *J*= 2.2 Hz, 1H), 2.81 (s, 3H);
MS (DCI, NH₃ + isobutane) m/z 391 (MH⁺, 100).

### ● Synthesis of xanthene (V) (R₁ = R₂ = R₄ = R₅ = OMe and R"₃ = Me)

### 1,3,9,11-Tetramethoxy-6-methyl-7H-14-oxa-benzo[α]naphthacen-8-ol 310 (V)

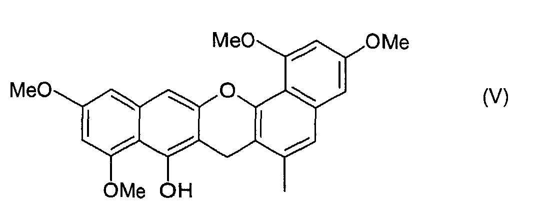

To a solution of dibenzoxanthone **302** (40.2 mg, 0.09 mmol) in anhydrous CH₂Cl₂ (9 mL) under argon, was added dropwise borane-methyl sulfide complex (86 µl, 0.9 mmol). The mixture was refluxed for 48 hours, and then allowed to cool to room temperature, diluted with aqueous HCl (0.5N), and extracted with CH₂Cl₂. After the usual workup, dibenzoxanthene **310 (V)** (38.8 mg, quantitative yield) was obtained, and used without further purification.
IR (neat) 3384, 3006, 2935, 2857, 1635, 1613 cm⁻¹;
¹H NMR (CDCl₃, 300 MHz) δ 9.46 (s, 1H), 7.18 (s, 1H), 6.93 (s, 1H), 6.61 (d, *J=* 2.2 Hz, 1H), 6.60 (d, *J=* 2.2 Hz, 1H), 6.47 (d, *J=* 2.2 Hz, 1H), 6.29 (d, *J=* 2.2 Hz, 1H), 4.01 (s, 3H), 4.00 (s, 3H), 3.97 (s, 2H), 3.87 (s, 3H), 3.86 (s, 3H), 2.43 (s, 3H);
¹³C NMR (CDCl₃, 75.4 MHz) δ 158.1 (C), 157.9 (C), 157.8 (C), 157.2 (C), 152.0 (C), 151.9 (C), 147.7 (C), 136.5 (C), 136.4 (C), 136.3 (C), 121.5 (CH), 113.0 (C), 110.9 (C), 107.2 (C), 103.7 (C), 102.4 (CH), 99.2 (CH), 98.8 (CH), 98.3 (CH), 96.1 (CH), 56.6 (CH₃), 56.2 (CH₃), 55.4 (CH₃), 55.3 (CH₃), 21.1 (CH₂), 19.9 (CH₃);
MS (DCI, NH₃+isobutane) m/z 433 (MH⁺, 100);
HRMS calcd for C₂₆H₂₄O₆: 432.1573. Found: 432.1576 (M⁺).

### ● Synthesis of hypoxyxylerone derivative Ib (R₁ = R₂ = R₄ = R₅ = OMe and R'₃ = Me)

### Trimethyl-(1,3,9,11-tetramethoxy-6-methyl-7H-14-oxa-benzo[α]naphthacen-8-yloxy)-silane (311)

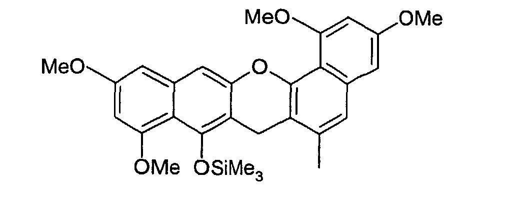

To a stirred solution of crude product **310** above (19.3 mg, 0.045 mmol) in anhydrous CH₂Cl₂ (0.9 mL) under argon at 0°C, were added dropwise 2,6-lutidine (52 µL, 0.45 mmol) and then trimethylsilyl trifluoromethanesulfonate (61 µL, 0.34 mmol). The reaction mixture was stirred for 3 hours, quenched by addition of saturated aqueous NH₄Cl and then extracted with dichloromethane. The organic layer was washed with saturated aqueous CuSO₄, and after the usual workup, silylated dibenzoxanthene **311** (22.5 mg, quantitative yield) is obtained as a green substance, which was used without further purification.
IR (neat) 3001, 2964, 2936, 2849, 1628, 1589, 1576 cm⁻¹;
¹H NMR (CDCl₃, 300 MHz) δ 7.19 (s, 1H), 7.10 (s, 1H), 6.61 (d, *J=* 2.3 Hz, 1H), 6.60 (d, *J=* 2.3 Hz, 1H), 6.48 (d, *J=* 2.3 Hz, 1H), 6.32 (d, *J=* 2.3 Hz, 1H), 4.02 (s, 3H), 4.01 (s, 3H), 3.99 (s, 2H), 3.87 (s, 3H), 3.86 (s, 3H), 2.42 (s, 3H), 0.28 (s, 9H);
¹³C NMR (CDCl₃, 75.4 MHz) δ 158.1 (C) 158.0 (C), 157.9 (C), 157.5 (C), 151.6 (C), 149.6 (C), 148.0 (C), 137.0 (C), 136.5 (C), 135.9 (C), 121.6 (CH), 113.5 (C), 112.7 (C), 111.0 (C), 110.1 (C), 105.6 (CH), 99.2 (CH), 98.3 (CH), 98.1 (CH), 96.9 (CH), 56.6 (CH₃), 55.4 (CH₃), 55.3 (CH₃), 55.2 (CH₃), 22.9 (CH₂), 19.9 (CH₃), 1.3 (CH₃);
MS (DCI, NH₃+isobutane) m/z 505 (MH⁺, 98), 447 (12), 433 (100), 431 (75), 417 (13).

### 1,3,9,11-Tetramethoxy-6-methyl-14-oxa-benzo[α]naphthacen-8-one 312 (Ib)

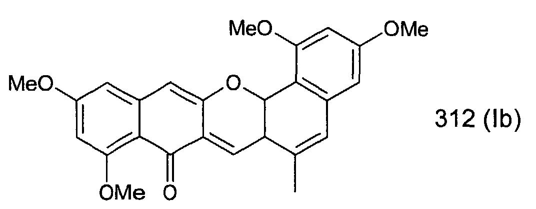

A solution of the above crude product **311** (22.5 mg, 0.045 mmol) and palladium (II) acetate (10.5 mg, 0.047 mmol) in anhydrous acetonitrile (1 mL) was refluxed under argon for 12 hours. After being allowed to cool to room temperature, the reaction mixture was filtered through a short pad of celite, concentrated in vacuo and the residue was immediately purified by flash column chromatography (silica gel treated with triethylamine 2.5 % w/v, dichloromethane and then dichloromethane-methanol 99:1) to give **312 (Ib)** as a blue substance (9.4 mg, 49 % for three steps).
IR (neat) 2967, 2930, 2858, 1663, 1613, 1591, 1530 cm⁻¹;
¹H NMR (CDCl₃, 300 MHz) δ 8.45 (d, *J=* 1.4 Hz, 1H), 7.15 (s, 1H), 6.58 (d, *J=* 2.2 Hz, 1H), 6.47 (d, *J=* 2.2 Hz, 1H), 6.42 (d, *J=* 2.2 Hz, 1H), 6.27 (d, *J*= 2.2 Hz, 1H), 6.24 (d, *J=* 1.4 Hz, 1H), 4.01 (s, 3H), 3.95 (s, 3H), 3.89 (s, 3H), 3.88 (s, 3H), 2.56 (s, 3H);
¹³C NMR (CDCl₃, 75.4 MHz) δ 178.9 (C), 164.5 (C), 164.2 (C), 161.4 (C), 159.7 (C), 151.1 (C), 144.2 (C), 140.0 (C), 134.7 (C), 134.2 (C), 133.7 (CH), 123.9 (C), 123.0 (CH), 114.9 (C), 112.6 (C), 109.5 (C), 102.3 (CH), 99.4 (CH), 99.2 (CH), 96.8 (CH), 95.4 (CH), 56.3 (CH₃), 56.1 (CH₃), 55.6 (CH₃), 55.5 (CH₃), 19.1 (CH₃);
MS (DCI, NH₃ + isobutane) m/z 431 (MH⁺, 100);
HRMS calcd for C₂₆H₂₂O₆ : 430.1416. Found : 430.1434 (MH⁺).

### Synthesis of products of formula (IVb) wherein (R₁ = R₂ = R₄ = R₅ = OH and R'₃ = Me); (R₁ = R₄ = R₅ = OSiEt₃, R₂ = OH and R'₃ = Me). Synthesis of products of products of formula (V) wherein (R₁ = R₄ = R₅ = OSiEt₃, R₂ = OH and R'₃ = Me); (R₁ = R₂, = R₄ = R₅ = OH and R'₃ = Me). Synthesis of products of formula (Ib) wherein (R₁ = R₂, = R₄= R₅ = OH and R'₃ = Me).

### 1,3,8,9,11-Pentahydroxy-6-methyl-14-oxabenzo[α]naphthacen-7-one 304 (IVb)

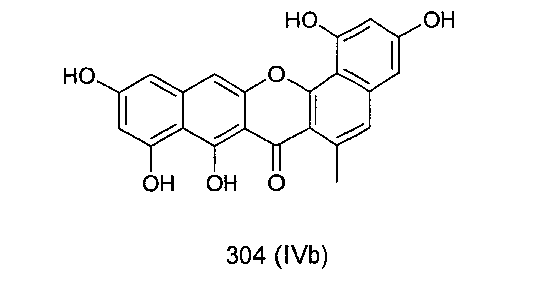

To a stirred suspension of dibenzoxanthone **302** (22.0 mg, 0.049 mmol) in anhydrous chloroform (1.5 mL) under argon at -50°C was added dropwise boron tribromide (740 µL of a 2 M freshly prepared solution in chloroform, 1.48 mmol). The mixture, which immediately turned purple, was allowed to warm slowly to room temperature, stirred in the dark for 24 hours, and then treated under argon at 0°C with several drops of methanol until the release of gaseous hydrobromic acid terminated. After dilution with saturated aqueous NaHCO₃ and water, the mixture was extracted with ethyl acetate. The aqueous phase was then acidified and extracted with ethyl acetate. The organic layers were combined and, after the usual workup, the crude product **304 (TVb)** (18.0 mg) was obtained as a brown solid, which was used without purification.
¹H NMR (CD₃COCD₃, 300 MHz) δ 7.11 (s, 1H), 6.69 (d, *J*= 2.2 Hz, 1H), 6.67 (s, 1H), 6.61 (d, *J=* 2.2 Hz, 1H), 6.45 (d, *J*= 2.2 Hz, 1H), 6.09 (d, *J*= 2.2 Hz, 1H), 2.81 (s, 3H);
MS (DCI, NH₃ + isobutane) m/z 391 (MH⁺, 100).

### 8,9-Dihydroxy-6-methyl-1,3,11-tris-triethylsilanyloxy-14-oxabenzo [α]naphthacen-7-one 320 (IVb)

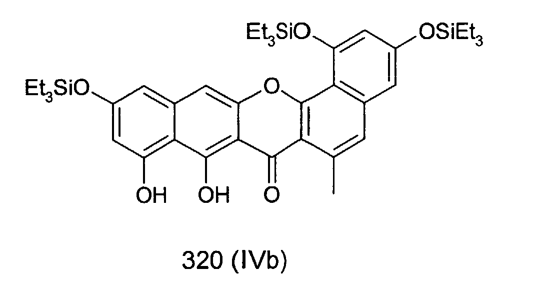

To a stirred solution of crude product **304** (18.0 mg) in anhydrous CH₂Cl₂ (1.5 mL) under argon at 0°C were added dropwise 2,6-lutidine (0.32 mL, 2.77 mmol) and then triethylsilyl trifluoromethanesulfonate (0.52 mL, 2.31 mmol). The reaction mixture was allowed to warm to room temperature, stirred for 24 hours, quenched by addition of saturated aqueous NH₄Cl, and then extracted with dichloromethane. The organic layer was washed with saturated aqueous CuSO₄ and, after the usual workup, the resultant crude product was purified by flash column chromatography (silica gel, pentane-EtOAc 99.5 : 0.5, 98 : 2, and then 9 :1) to give trisilylated dibenzoxanthone **320 (IVb)** (12.4 mg, 30 % for two steps) as a brown solid.
IR (neat) 3390, 2957, 2923, 2848, 1640, 1619, 1598 cm⁻¹;
¹H NMR (CDCl₃, 300 MHz) δ 9.99 (s, 1H), 7.16 (s, 1H), 7.02 (s, 1H), 6.74 (d, *J=* 2.2 Hz, 1H), 6.62 (d, *J=* 2.2 Hz, 1H), 6.53 (d, *J=* 2.2 Hz, 1H), 6.39 (d, *J=* 2.2 Hz, 1H), 2.89 (s, 3H), 1.09-0.73 (m, 45H);
¹³C NMR (CDCl₃, 75.4 MHz) δ 184.0 (C), 164.6 (C), 159.5 (C), 158.1 (C), 155.8 (C), 151.1 (C), 140.7 (C), 140.5 (C), 135.9 (C), 131.0 (C), 129.0 (C), 124.8 (CH), 112.8 (C), 111.9 (C), 111.1 (CH), 108.8 (CH), 106.7 (C), 106.2 (CH), 103.8 (CH), 102.9 (C), 100.1 (CH), 23.9 (CH₃), 6.9 (CH₃), 6.8 (CH₃), 6.7 (CH₃), 5.4 (CH₂), 5.3 (CH₂), 5.2 (CH₂);
MS (DCI, NH₃+isobutane) m/z 733 (MH⁺, 100).

### 6-Methyl-1,3,11-tris-triethylsilanyloxy-7H-14-oxabenzo[a]naphthacen-8,9-diol 321 (V)

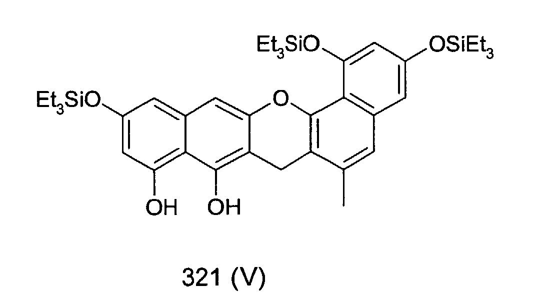

To a solution of dibenzoxanthone **320** (12.4 mg, 0.017 mmol) in anhydrous CH₂Cl₂ (0.8 mL) under argon was added dropwise borane-methyl sulfide complex (16 µl, 0.17 mmol). The mixture was refluxed for 24 hours, allowed to cool to room temperature, diluted with saturated aqueous NH₄Cl, and then extracted with CH₂Cl₂. After the usual workup, dibenzoxanthene **321 (V)** (12.1 mg, quantitative yield) was obtained as a brown substance and used without further purification.
¹H NMR (CDCl₃, 300 MHz) δ 7.14 (s, 1H), 6.98 (s, 1H), 6.70 (d, *J=* 2 Hz, 1H), 6.66 (d, *J=* 2 Hz, 1H), 6.45 (d, *J=* 2 Hz, 1H), 6.37 (d, *J=* 2 Hz, 1H), 4.00 (s, 2H), 2.40 (s, 3H), 1.09-0.71 (m, 45H);
MS (DCI, NH₃+isobutane) m/z 719 (MH⁺, 3), 391 (7), 124 (42), 110 (100).

### 6-Methyl-7H-14-oxabenzo[α]naphthacene-1,3,8,9,11-pentaol 322 (V)

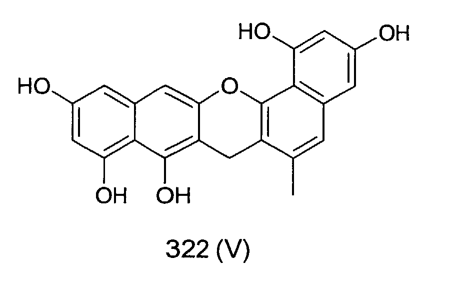

Dibenzoxanthene **321** (12.1 mg, 0.017 mmol) was stirred in aqueous HCl (1 N)-methanol 1 :1 (5 mL) at room temperature for 30 minutes. The mixture was partially concentrated in vacuo, washed with chloroform to remove silylated byproducts, and then extracted with ethyl acetate. After the usual workup, dibenzoxanthene **322 (V)** (6.3 mg) was obtained as a brown substance.
¹H NMR (CD₃COCD₃, 300 MHz) δ 7.19 (s, 1H), 6.95 (s, 1H), 6.68 (d, *J=* 2 Hz, 1H), 6.63 (d, *J=* 2,3 Hz, 1H), 6.47 (d, *J=* 2,3 Hz, 1H), 6.44 (d, *J=* 2 Hz, 1H), 3.94 (s, 2H), 2.42 (s, 3H).

### 1,3,9,11-Tetrahydroxy-6-methyl-14-oxabenzo[α]naphthacen-8-one 325 (Ib)

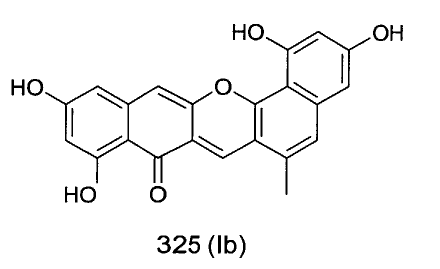

Dibenzoxanthene 322 (6.1 mg) was submitted to air oxidation.

The product 325 (Ib) was purified by inverse phase column chromatography using a microbondapack C18 column, length 30 cm, diameter 3.9 mm eluting the compound with a 0 to 100 % methanol in water solution at pH 2.5 (phosphoric acid) at a flow rate of 2 ml/mn with a detector marketed by Waters (UV 996 plate diode array)
UV λmax 235.6, 304.1, 325.4, 351.6, 419.7, 660.0 nm.
MS (NICI) 374 (M^{• -})

### Biological tests

Supercoiled pKMp27 DNA (0.5 µg) was incubated with 6 units of topo I at 37°C for 45 minutes in relaxation buffer (50 mM TRIS, pH 7.8, 50 mM KCL, 10 mM MgCl2, 1 mM Dithiothreitol, 1mM EDTA) in the presence of varying concentrations of the drug under study. Reactions were terminated by adding SDS to 0.25 % and proteins K to 250 µg/mL. DNA samples were then added to the electrophoresis dye mixture (3µL) and electrophoresed in a 1 % agarose gel at room temperature for 3 hours. Gel were stained with ethydium bromide (1mg/mL), washed and photographed under UV light. Similar experiments were performed using ethydium-containing agarose gels.

In the presence of topoI inhibitor, the nicked DNA fraction is significantly increased in a dose-dependent manner, reflecting the stabilization of TopoI-DNA complexes.

The products of the present invention were found active at a dosage of 0.5µg

## Claims

1. Process of manufacture of hypoxyxylerone derivatives of formula (Ia) 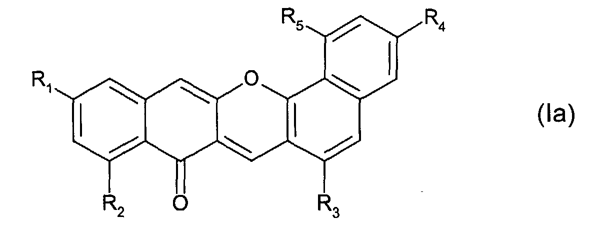 in which R₁, R₂, R₄, R₅ which may be the same or different from H, OH, NH₂, NHR, NR₂, OR, R wherein R represents an alkyl group bearing 1 to 6 carbon atoms in a straight or ramified chain eventually substituted by a group such as OH, OR', NH₂, NHR', NR'₂ wherein R' represents a straight, ramified, or cyclic alkyl chain bearing 1 to 6 carbon atoms, eventually forming an heterocyclic ring by including the heteroatom O or N, and R₃ represents a CH₂OH or CH₂OR substituent wherein R is defined as above, with a disclaimer concerning hypoxyxylerone itself,
**characterized in that**
1) a 3-protected hydroxy naphthalene 2-carboxylic acid of formula (IIa), 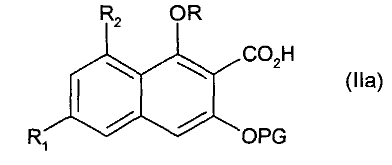 wherein R₁, R₂ and R have the same meaning as for formula (Ia) except OH or NH₂ or NHR and PG is a protective group, is condensed with a 4,5,7 tri-substituted 3-halogeno naphthalene of formula (Iib) 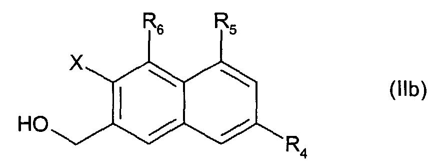 wherein X = halogen and R₄-R₅ have the meaning of formula (Ia) except OH, NH₂, NHR and R6 is an alkoxy group bearing 1 to 6 carbon atoms to give a product of formula (II), 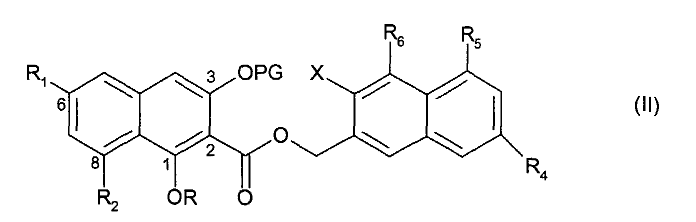 in which
X represents a halogen
PG represents a protective group
the product of formula (II) is reacted with an organoalkali metal derivative to give a product of formula (III) 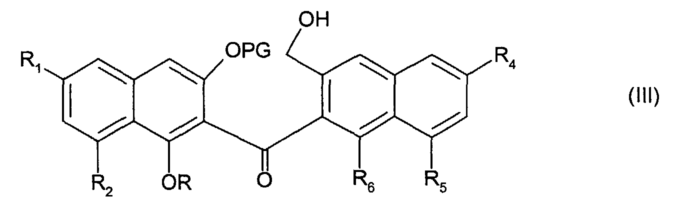
2) the OH of the hydroxymethyl group is protected to give CH₂OR
the OPG group is cleaved to give an OH group
the cycle is closed by reaction with a base thus linking with an oxygen atom the carbons that bore the OPG and R₆ groups,
the OH in the α-position to the keto group is deprotected to give a product of formula (IVa), 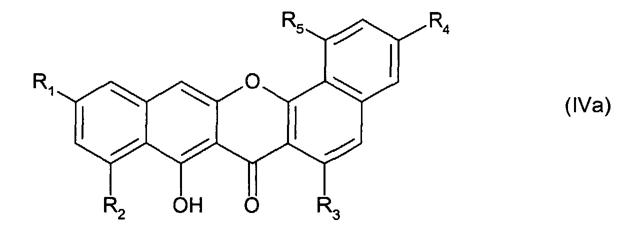
3) the keto group is reduced to give a product of formula (Va) 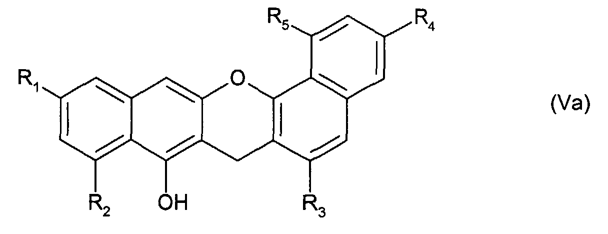
4) the OH in the α position to the methylene group is protected and the product is then oxidized to give a product of formula (Ia), R₁-R₅ are deprotected.

2. Process according to claim 1 in which the condensation of step 1 is carried out in the presence of diethyl azodicarboxylate and a phosphine derivative.

3. Process according to claim 2 where the phosphine derivative is a triarylphosphine.

4. Process according to claim 1 in which step 1 is carried out in presence of butyllithium.

5. Process according to claim 1 step 2 in which the protection of the hydroxymethyl group is carried out with silver oxyde and iodomethane, sodium hydride and iodomethane, or methyl triflate or a salt of trimethyloxonium and a pyridine derivative.

6. Process according to claim 1 step 2 in which the product obtained from claim 5 is deprotected (OPG) by hydrogenation with palladium hydroxide or palladium on carbon or by treatment with ammonium formate in the presence of palladium on carbon.

7. Process according to claim 1 step 2 in which the cycle is closed by action of an alkaline base.

8. Process according to claim 1 step 2 in which the product of claim 7 is deprotected at the OH group α to the keto group by action of lithium chloride, boron trichloride, or zinc and sodium hydroxide.

9. Process according to claim 1 step 3 in which the product of claim 8 is reduced.

10. Process according to claim 9 where the reduction is carried out in presence of borane-dimethylsulfide complex.

11. Process according to claim 1 step 4 in which the product of claim 10 is protected.

12. Process according to claim 11 where the protection is carried out by a silyl group.

13. Process according to claim 1 step 4 where the product of claim 12 is oxidized by action of a palladium derivative.

14. Process of manufacture of hypoxyxylerone derivatives of formula (Ib) 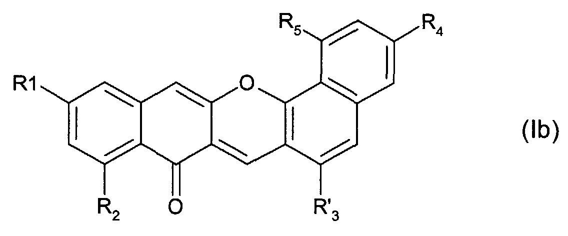 in which R₁, R₂, R₄ and R₅ have the same meaning as in claim 1, and R'₃ represents a hydrogen or an R substituent, wherein R has the same meaning as above in claim 1 **characterized in that**
1) a 2-alkoxycarbonylmethyl benzoic acid ester or a derivative of it of formula (VI) 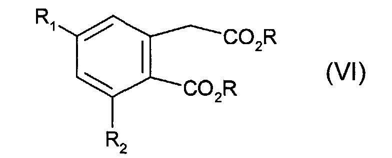 is condensed with a 1-(1-hydroxy, 1-hydroxy-3-alkyl or 1-hydroxy-3-alkoxymethyl naphthalene-2yl) ethanone or a derivative of it of formula (VII) 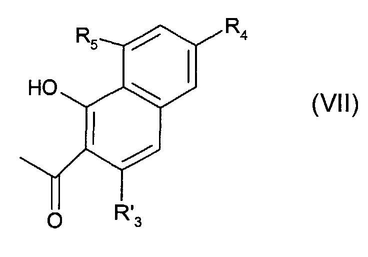 wherein R₁, R₂, R'₃, R₄ and R₅ have the same meaning as in claim 14 except OH, NH₂, NHR
in presence of a base to obtain a product of formula (IVb), 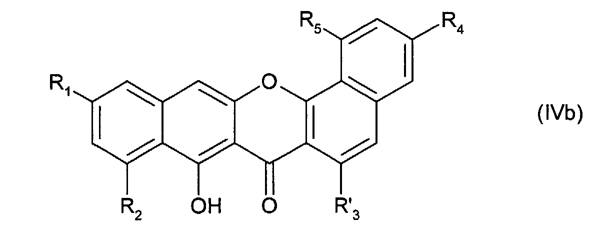 wherein R₁, R₂, R'₃, R₄ and R₅ have the same meaning as in claim 14
2) the keto group is reduced
3) the OH in the α position to the methylene group is protected and the product is then oxidized to give a product of formula (Ib).

15. Process according to claim 14, step 2, where the reduction is carried out in the presence of borane-dimethylsulfide complex.

16. Process according to claim 14 step 3 in which the product is protected by a silyl group.

17. Process according to claim 14 step 3 where the product is oxidized by action of a palladium derivative.

18. Process according to claim 14 wherein steps 2-3 are replaced by the following steps :
2') the hydroxyl protecting group(s) is(are) cleaved
3') silylation is performed
4') the keto group is reduced
5') the silyl group(s) is(are) hydrolysed
6') the product is oxidized to give a product of formula (Ib).

19. Process according to claim 18 step 2', where the product of formula (IVb) is deprotected with boron tribromide.

20. Process according to claim 18 step 3', where the product is silylated with triethylsilyl trifluoromethanesulfonate.

21. Process according to claim 18 step 4', where the product is reduced in the presence of borane-dimethyl sulfide complex.

22. Process according to claim 18 step 5', where the product is hydrolysed with aqueous chlorhydric acid in methanol.

23. Process according to claim 18 step 6', where the product is oxidized by air.

24. Product of formula (Ia) 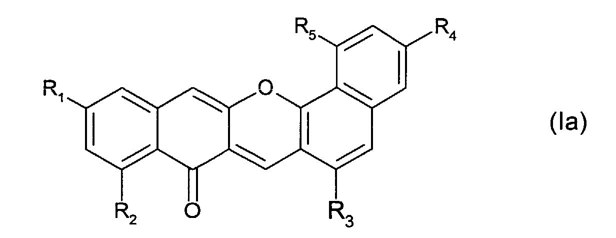 in which R₁, R₂, R₄, R₅ may be the same or different from H, OH, NH₂, NHR, NR₂, OR, R, wherein R represents an alkyl group bearing 1 to 6 carbon atoms in a straight or ramified chain eventually substituted by a group such as OH, OR', NH₂, NHR', NR'₂ wherein R' represents a straight, ramified, or cyclic alkyl chain bearing 1 to 6 carbon atoms, eventually forming an heterocyclic ring by including the heteroatom O or N, and R₃ represents a -CH₂OH or -CH₂OR substituent wherein R is defined as above with a disclaimer concerning hypoxyxylerone itself.

25. Products of formula (Ib) 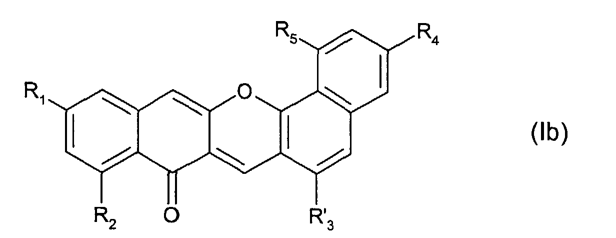 in which R₁, R₂, R₄, R₅ which may be the same or different from H, OH, NH₂, NHR, NR₂, OR, R wherein R represents an alkyl group bearing 1 to 6 carbon atoms in a straight or ramified chain eventually substituted by a group such as OH, OR', NH₂, NHR', NR'₂ wherein R' represents a straight, ramified or cyclic alkyl chain bearing 1 to 6 carbon atoms, eventually forming an heterocyclic ring by including the heteroatom O or N, and R'₃ represents a hydrogen or an R substituent, wherein R has the same meaning as above.

26. Product of formula (IV) 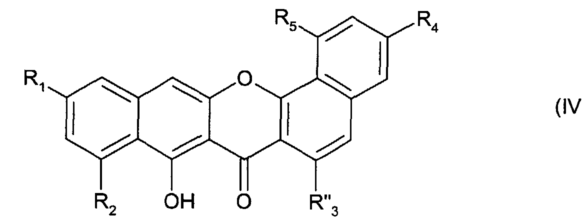 in which R₁, R₂, R₄, R₅ have the same meaning as above in formula (Ia) or (Ib) and wherein R"₃ represents a -CH₂OH or -CH₂OR substituent or an hydrogen or an R substituent.

27. Product of formula (V) 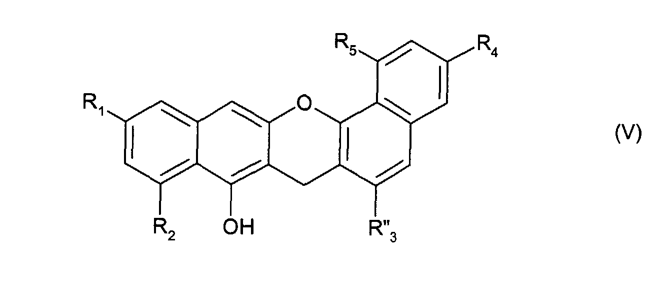 in which R₁, R₂, R₄, R₅ have the same meaning as in formula (Ia) or (Ib) and wherein R"₃ represents a -CH₂OH or -CH₂OR substituent or an hydrogen or an R substituent.

28. Use of products of claims 24 to 25 as a medicine including the hypoxyxylerone itself.

29. Use of products of claim 26 wherein at least one of R₁, R₂, R₄ or R₅ is OH as a medicine.

30. Use of any of the products of claim 28 or 29 as a medicine for treating cancer.

31. Use of products of claims 24 to 25 for the preparation of a medicine for treating cancer.
